# EUROPEAN PATENT SPECIFICATION

(11) **EP 2 167 674 B1**
(45) Date of publication and mention of the grant of the patent: **18.04.2018**
(21) Application number: 08775485.9
(22) Date of filing: 13.06.2008
(51) Int. Cl.: A23K 20/163, A23L 29/30, A61K 31/715, A61K 36/064, A61P 1/00, C07H 1/08, C12P 19/04

(54) **NUTRITIONAL COMPOSITIONS**
ERNÄHRUNGSZUSAMMENSETZUNG
COMPOSITIONS NUTRITIONNELLES

(30) Priority: 13.06.2007 FI 20070471
(43) Date of publication of application: 31.03.2010
(73) Proprietor: Glykos Finland Oy, 00790 Helsinki (FI); Hankkija Oy, 05800 Hyvinkää (FI)
(72) Inventor: NIEMELÄ, Ritva, FI-00730 Helsinki (FI); SAARINEN, Juhani, FI-00370 Helsinki (FI); HELIN, Jari, FI-05200 Rajamäki (FI); WAKKINEN, Janica, FI-00790 Helsinki (FI)
(74) Representative: Papula Oy
(86) International application number: PCT/FI2008/050360
(87) International publication number: WO 2008/152207

(56) References cited:
- WO-A1-97/02356
- WO-A1-98/27829
- WO-A1-2006/067145
- WO-A1-2006/121803
- GB-A- 2 048 918
- JP-A- 2004 099 580
- US-A1- 2002 061 345
- US-A1- 2006 014 717
- DATABASE WPI Derwent Publications Ltd., London, GB; AN 1994-277411[34], XP008117457 & RU 2 007 928 C1 (MOSC APPLIED BIOTECH INST.) 28 February 1994

## Description

The present invention is directed to a novel saccharide composition isolated from yeast culture. The invention is also directed to yeast cell based saccharide composition with increased water-solubility. The saccharide compositions disclosed in the invention can be used as nutritional or pharmaceutical additives or as part of nutritional or pharmaceutical composition in order to promote the health of animal or human subject to which said composition is administered. The invention is also directed to methods of producing said saccharide compositions.

### BACKGROUND OF THE INVENTION

Progut™ is whole yeast-based animal feed ingredient for maintaining good gut health, improving growth, and replacing antibiotics in most gastrointestinal problems in animals (see US 20020061345). Progut™ is produced from whole brewery yeast (*Saccharomyces cerevisiae*) and therefore it contains both yeast cell wall particles (mostly mannoproteins and β-glucans) and internal cell nucleotides. All these components have been shown to be important for the activity of Progut™.

Patent application WO 98/27829 discloses a procedure for preparing a food additive, in which a raw material based on a vegetable, animal and/or microbial product and containing oligosaccharides and/or polysaccharides is treated hydrolytically so that the cell wall structure is opened and the use of the food additive for the prevention of gastric disorders and intestinal diseases.
US 2002/061345 discloses a procedure for preparing a food additive, inbrewing yeast raw material containing oligosaccharides and/or polysaccharides is filtered and treated hydrolytically so that the cell wall structure is opened and the use of the food additive for the prevention of gastric disorders and intestinal diseases.
WO 2006/121803 discloses methods for producing yeast &bgr;-glucan and mannan preparations. The methods employ an autolysis process, followed by an enzymatic treatment using a high-PH protease or a high-PH protease followed by a treatment with an enzyme comprising at least one of an amylase, lipase and a combination thereof.
RU 2007928 discloses a production of yeast extracts from yeast biomass, by two-stage treatment with hydrochloric acid solutions, neutralising and separation to produce two types of yeast extract.
JP 2004 099580 discloses a immunity-reinforcing composition which is obtained by self-digesting the yeast, adding an enzyme containing a protease and a beta-1,3-glucanase simultaneously while self-digesting, or adding after the self digestion, for improving the immune-activating activity of the yeast.

The present invention reveals novel progut type products especially produced with optimized strong acid hydrolysis or enzymatic hydrolysis conditions or surprisingly effective combinations thereof to obtain novel bioactive and highly soluble product. It was observed that surprisingly strong acid treatment conditions, including very high acid concentrations including about 0.5 M to about 1 M acid and/or high reaction temperature in range of 75- 100 degrees of Celsius, were needed to obtain increased solubilization and obtaining highly bioactive low molecular weight polysaccharides and oligosaccharides. The conditions are drastic and reveal high resistance of yeast materials to hydrolysis, however there is optimum with the conditions so that increase of acid to 2 M at high temperature causes less effective solubilization of the cell wall materials and/or increased amount of free monosaccharides caused by hydrolysis closing to total hydrolysis.

The unique chemical feature including high mannose oligosaccharide and low starch oligosaccharide content and high Glcβ-saccharide content are related to high bioactivity of the novel saccharide fractions. These are clearly different from prior products and competing products. The optimal hydrolysis conditions to produce substantial amount of effective mannose oligosaccharides and Glcβ-saccharides to same preparation is novel and inventive especially when the saccharides are produced to same preparation and from yeast raw material including native yeast without major hydrolytic or extraction chemical treatments such as fermentation derived yeast such as brewery process derived yeast.

It is realized that acid treatments of yeast materials in background are mainly mild treatments with low acid concentrations at modest pH ranges and reaction temperatures, are typically used together with alkaline treatments to obtain mainly insoluble cell wall preparations referred as glucans. There is no evidence of glucan preparation acid derived beta6

In this invention a set of old and new Progut™ samples as well as the samples from selected related products are analyzed. The analysis is performed mainly by gel permeation chromatography, but the process uses NMR analysis of the soluble components isolated here by gel permeation chromatography.

Another aim of this study was to produce a new Progut™ with increased solubility and without loosing its activity. This might be achieved by degrading the mannoproteins and / or β-glucans either enzymatically, mechanically, or by acid hydrolysis.

### BRIEF DESCRIPTION OF THE DRAWINGS

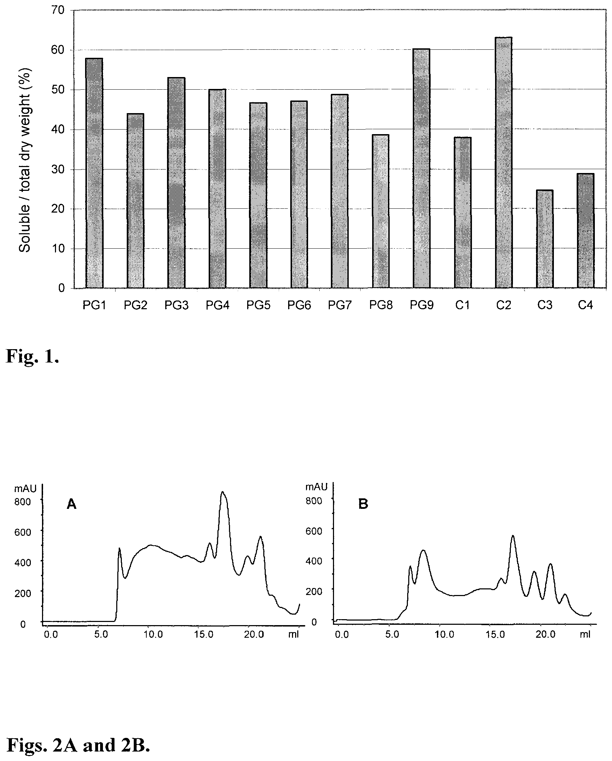

**Fig. 1****. The amounts of soluble components in Progut-type product (PG) and related samples.**
   PG1-PG9, Progut-type product samples; related samples: C1, Agrimos; C2, Ascogen; C3, Alphamune Alpharma; C4, Bio-Mos.
**Fig. 2****. Comparison of PG1 and PG2, from 0.2% solution.** Gel permeation chromatograms of **A.** PG1 and **B.** PG2 water soluble components from 0.2% solutions (equal sample preparations); in Superdex Peptide column (A₂₁₄ was measured).

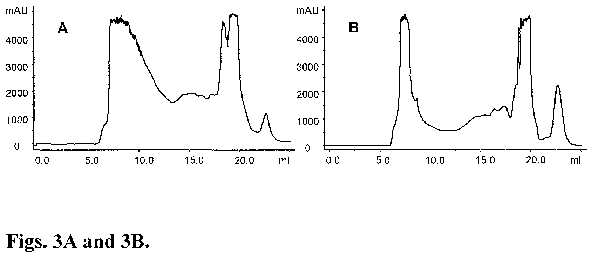

**Fig. 3****. Comparison of PG1 and PG2, from 1% solution.** Gel permeation chromatograms of **A.** PG1 and **B.** PG2 water soluble components from 1% solutions (equal sample preparations); in Superdex Peptide column (A₂₁₄ was measured).

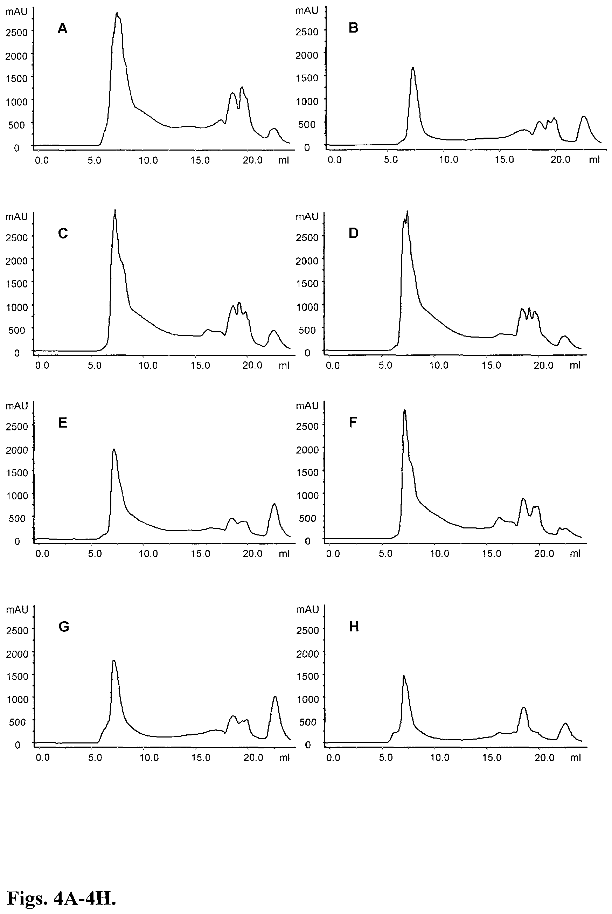

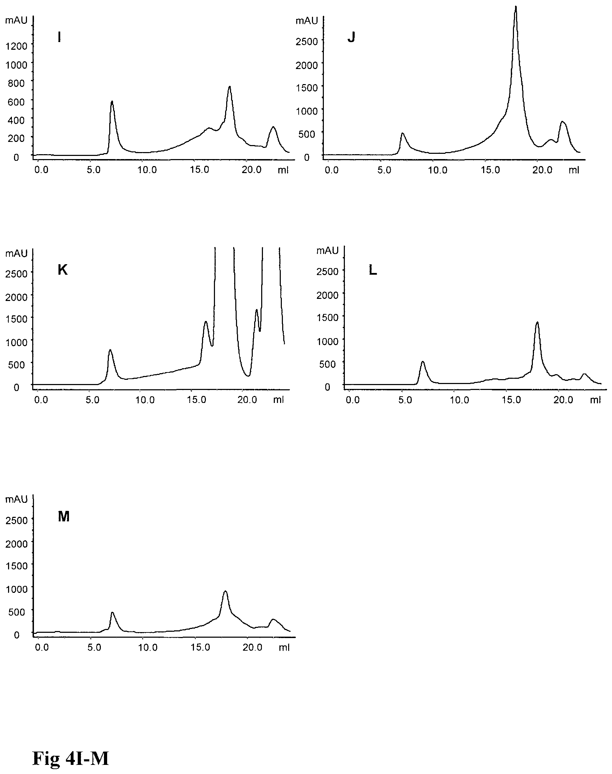

**Fig. 4****. Comparison of nine PG samples and related samples.** Gel permeation chromatograms of **A.** PG1, **B.** PG2, C. PG3, **D.** PG4, **E.** PG5, **F.** PG6, **G.** PG7, **H.** Pug8, **I.** PG9, **J.** C1 **K.** C2, **L.** C3, **M.** C4, water soluble components from 1% solutions (equal sample preparations); in Superdex Peptide column (A₂₃₀ was measured). With PG9, approximately 50% less material was analyzed. However, this is compensated by adjusting the scale accordingly in order to be able to compare the results equally.

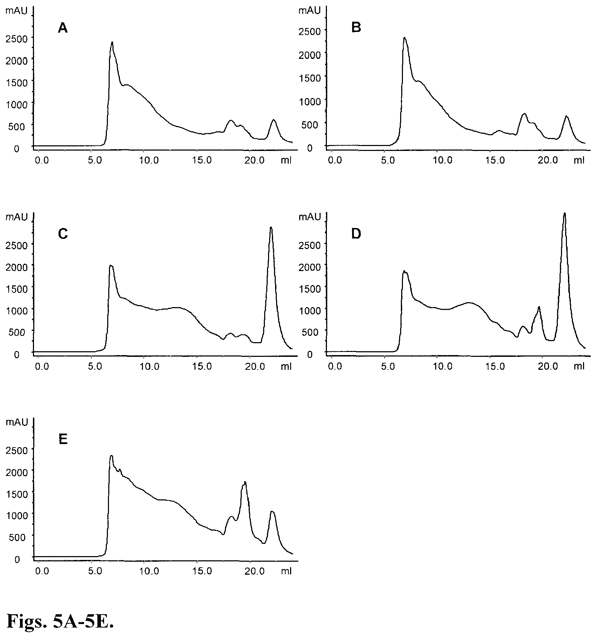

**Fig. 5****. Comparison of five PG samples.** Gel permeation chromatograms of **A.** PG10, **B.** Pug11, **C.** PG12, **D.** PG13, **E.** PG14, water soluble components from 1% solutions (equal sample preparations); in Superdex Peptide column (A₂₃₀ was measured).

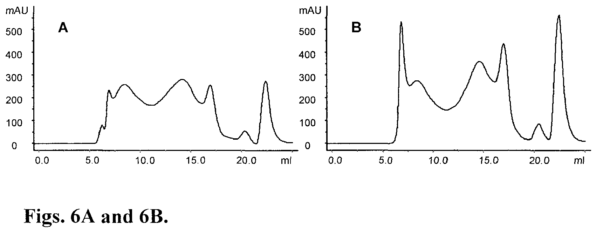

**Fig. 6****. Comparison of PG samples generated from brewery yeast solution and its homogenized version.** Gel permeation chromatograms of acid hydrolyzed **A.** brewery yeast and **B.** homogenized brewery yeast water soluble components from 1% solutions (equal sample preparations); in Superdex Peptide column (A₂₁₄ was measured).

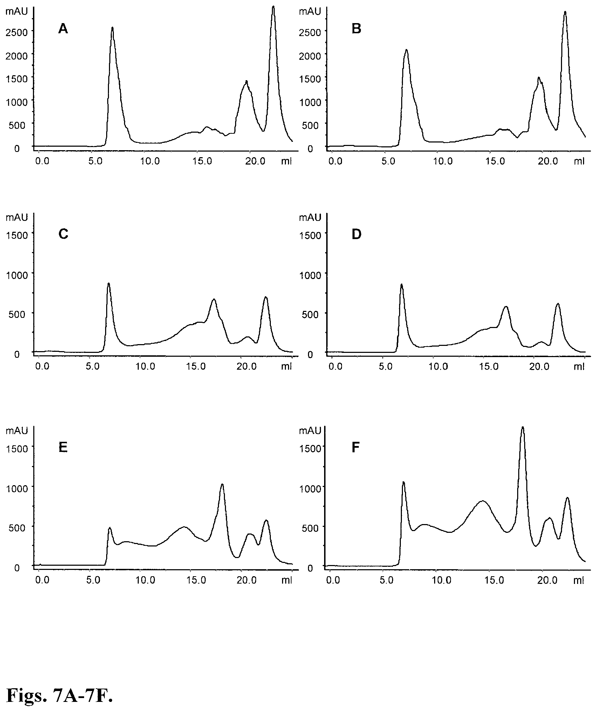

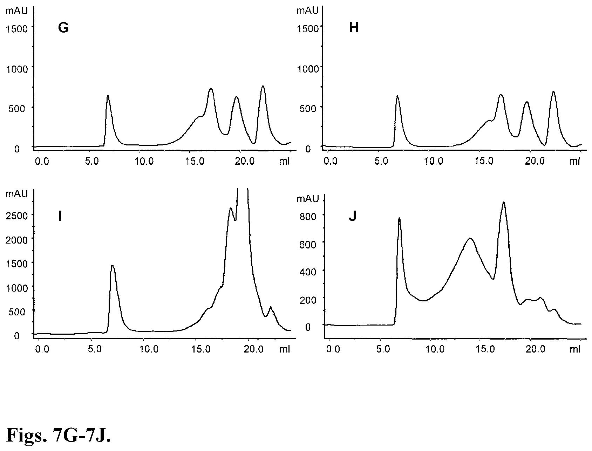

**Fig.7****. Comparison of samples generated by enzymatic or chemical degradations for bacterial adhesion tests.** Gel permeation chromatograms of **A.** Brewery yeast (BY) + glucanex (G), **B.** Homogenized BY (HBY) + G, **C.** BY G + savinase (S), **D.** HBY G+S, **E.** BY 1M H₃PO₄, **F.** HBY 1M H₃PO₄, **G.** BY G + pectinase (PE), **H.** HBY G+PE, **I.** PG1 PE, **J.** PG1 + PR, water soluble components from 1% solutions (equal sample preparations); in Superdex Peptide column. A, B, and I are preparative chromatograms, where A₂₃₀ was measured (comparable with each other); the others were analytical, where A₂₁₄ was measured (comparable with each other).

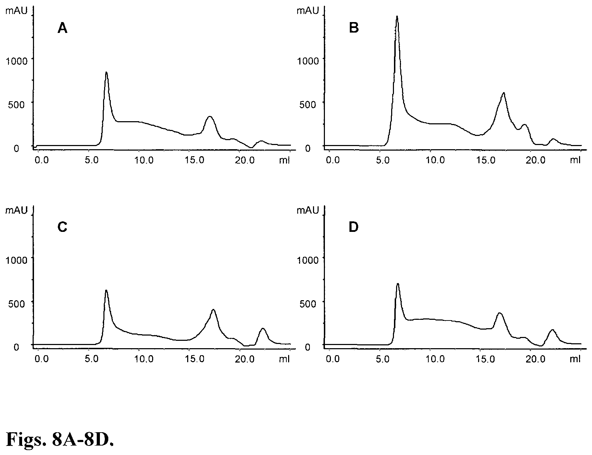

**Fig. 8****. Comparison of PG1, water-soluble PG (PG WS), water-soluble PG with emulgator (PG WS+E), and overhydrolyzed PG.** Gel permeation chromatograms of **A.** Pug1, **B.** PG WS, **C.** PG WS+E and **D.** PG 15h water soluble components from 1% solutions (equal sample preparations); in Superdex Peptide column (A₂₁₄ was measured).

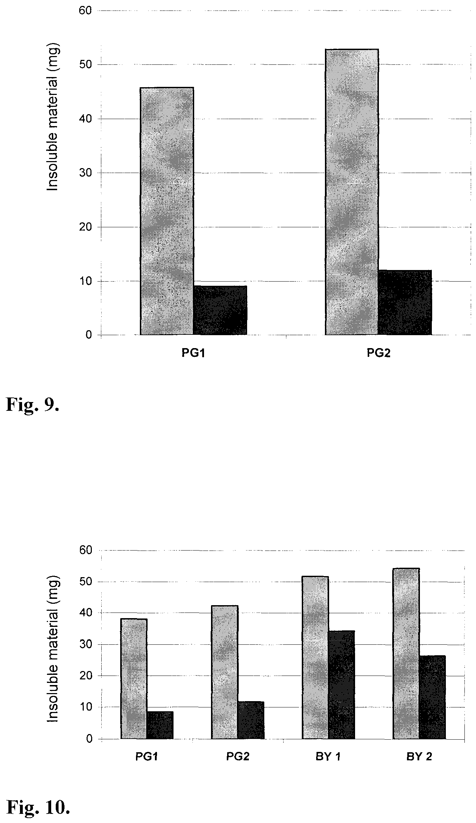

**Fig. 9****. Effect of pronase on Progut-type product samples.** Progut-type product samples 1 and 2 (PG1 and PG2) without (grey columns) and with (dark grey columns) pronase digestion.
**Fig. 10****. Effect of pronase on Progut-type product and brewery yeast solution.** Progut-type product samples 1 and 2 (PG1 and PG2) without (grey columns) and with (dark grey columns) pronase digestion (second experiment). Brewery yeast (BY) was treated either first with pronase and then with acid hydrolysis (BY1) or first with acid hydrolysis and then with pronase (BY2), dark grey columns. As controls BY was hydrolyzed by acid with the process procedure (grey columns).

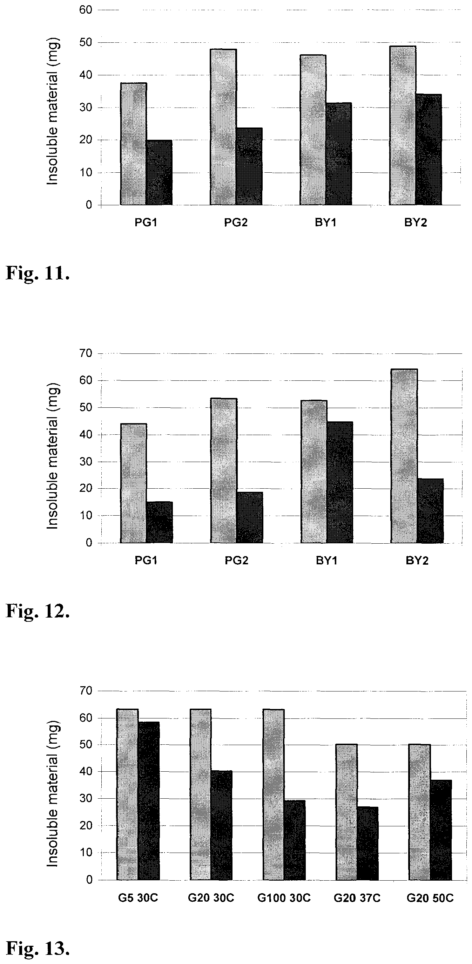

**Fig. 11****. Effect of savinase on Progut-type product and brewery yeast solution.** Progut-type product samples 1 and 2 (PG1 and PG2) without (grey columns) and with (dark grey columns) savinase digestion. Brewery yeast (BY) was treated either first with savinase and then with acid hydrolysis (BY1) or first by acid hydrolysis and then with savinase (BY2), dark grey columns. As controls BY1 and BY2 were hydrolyzed by acid with the process procedure (grey columns).
**Fig. 12****. Effect of pectinase on Progut-type product and brewery yeast solution.** Progut-type product samples 1 and 2 (PG1 and PG2) without (grey columns) and with (dark grey columns) pectinase digestion. Brewery yeast (BY) was treated either first with pectinase and then with acid hydrolysis (BY1) or first by acid hydrolysis and then with pectinase (BY2), dark grey columns. As controls BY1 and BY2 were hydrolyzed by acid with the process procedure (grey columns).
**Fig. 13****. Effect of glucanex on brewery yeast solution.** First experiment, brewery yeast with glucanex 5, 20, 100 mg at 30°C (G5 30C, G20 30C, G100 30C, respectively), after which the process acid treatment, dark grey columns. Second experiment, brewery yeast with 20 mg of glucanex at 37 or 50°C (G20 37C, G20 50C, respectively), after which the process acid treatment, dark grey columns. As control, in both experiments the brewery yeast was treated only with the process acid treatment, grey columns.

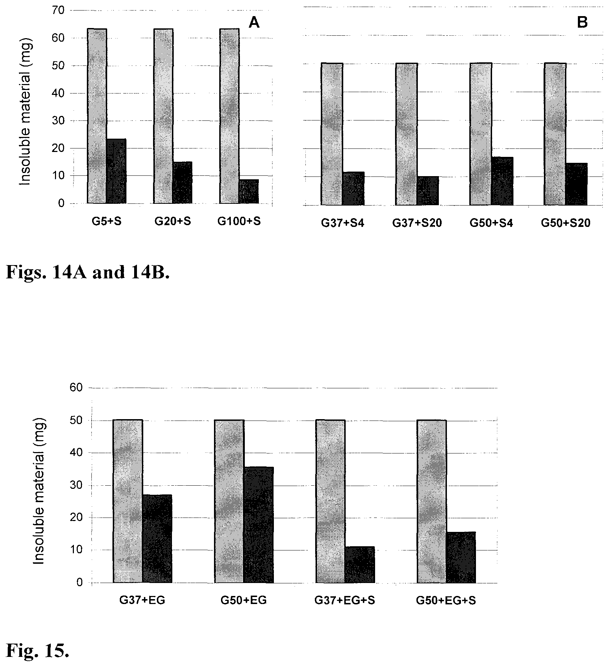

**Fig. 14****. Combined effect of glucanex and savinase on brewery yeast solution. A.** Brewery yeast solution first with glucanex 5, 20, 100 mg at 30°C and then with savinase 4 µl at 37°C (G 5+S, G 20+S, G 100+S, respectively), after which the process acid treatment, dark grey columns. **B.** Brewery yeast solution first with glucanex 20 mg at 37°C and then with savinase 4 or 20 µl at 37°C (G 37+S4, G 37+S20, respectively), dark grey columns, or with glucanex 20 mg at 50°C and then with savinase 4 or 20 µl at 37°C (G 50+S4, G 50+S20, respectively), after which the process acid treatment, dark grey columns. As controls for both **A.** and **B.**, brewery yeast solution was incubated without enzymes and treated only with the process acid treatment, grey columns.
**Fig. 15****. Combined effect of glucanex and endoglucanase and / or savinase on brewery yeast solution.** Brewery yeast solution, with glucanex 20 mg and endoglucanase 200 µg at 37°C or 50°C (G 37+EG, G 50+EG, respectively) or first with glucanex 20 mg, endoglucanase 200 µg at 37°C or 50°C and then with savinase 4 µl at 37°C (G 37+EG+S, G 50+EG+S, respectively) followed by the process acid treatment, dark grey columns. As controls, brewery yeast solution was incubated without enzymes and treated only with the process acid treatment, grey columns.

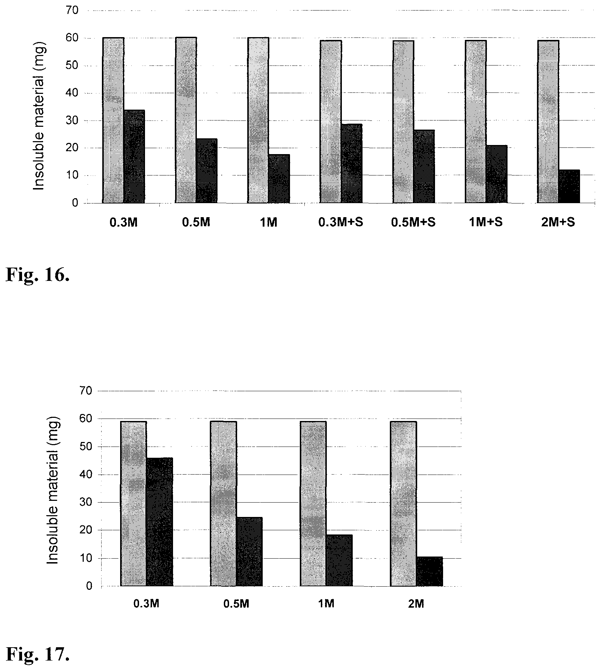

**Fig. 16****. Effect of higher concentrations of H₃PO₄ combined with savinase treatment on brewery yeast solution.** Acid hydrolysis of brewery yeast solution with different H₃PO₄-concentrations, 0.3M, 0.5M, 1M at 100°C (0.3M, 0.5M, 1M, respectively), dark grey columns. Incubation of brewery yeast solution with savinase (4 µl), then acid hydrolysis with different H₃PO₄-concentrations, 0.3M, 0.5M, 1M, 2M at 80°C (0.3M+S, 0.5M+S, 1M+S, respectively), dark grey columns. As controls, brewery yeast solution was treated with the process acid treatment, grey columns.
**Fig. 17****. Effect of HCl on brewery yeast solution.** Acid hydrolysis of brewery yeast solution with different HCl concentrations, 0.3M, 0.5M, 1M, 2M at 80°C (0.3M, 0.5M, 1M, 2M respectively), dark grey columns. As control, brewery yeast solution was treated with the process acid treatment, grey columns.

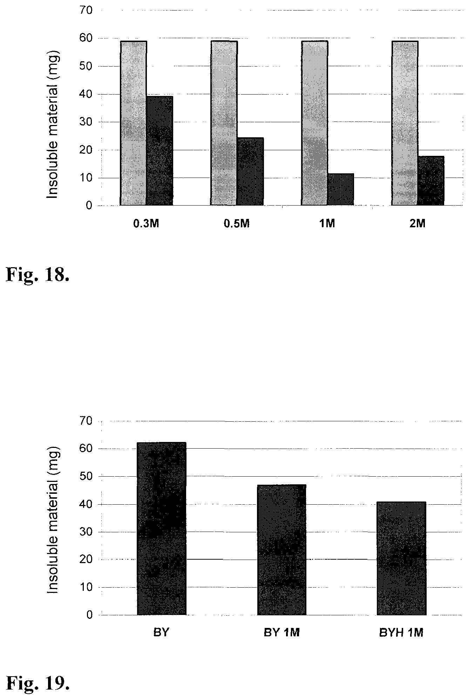

**Fig. 18****. Effect of H₂SO₄ on brewery yeast solution.** Acid hydrolysis of brewery yeast solution with different H₂SO₄ concentrations, 0.3M, 0.5M, 1M, 2M at 80°C (0.3M, 0.5M, 1M, 2M respectively), dark grey columns. As control, brewery yeast solution was treated with the process acid treatment, grey columns.
**Fig. 19****. Effect of homogenization.** Brewery yeast solution was treated with process acid hydrolysis or 1 M H₃PO₄, and the homogenized brewery yeast solution was treated with 1 M H₃PO₄, at 80°C (BY, BY 1M, HBY 1M, respectively).

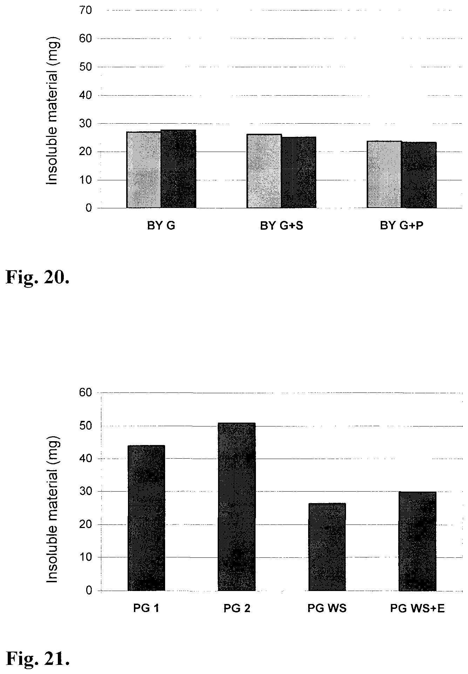

**Fig. 20****. Effect of homogenization with glucanex, savinase and pectinase.** Brewery yeast solution (grey) and its homogenized version (dark grey), treated with glucanex, glucanex and savinase, and glucanex and pectinase (BY G, BY G+S, BY G+P, respectively), followed by process acid hydrolysis.
**Fig. 21****. Comparison between the solubilities of PG 1, PG 2, PG WS and PG WS+E.**

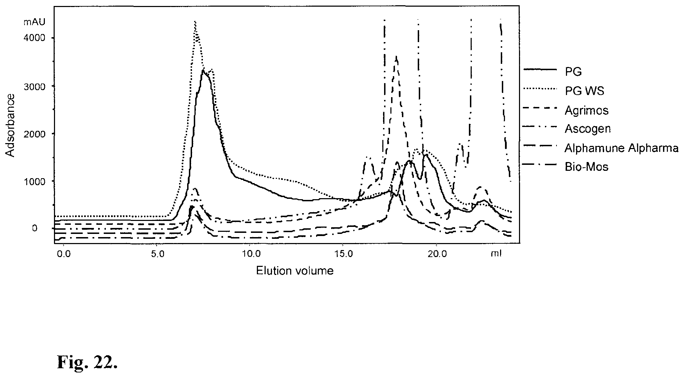

**Fig. 22****. Comparison of PG1, PG-WS, Agrimos, Ascogen, Alphamune Alpharma, and Bio-Mos by gel permeation chromatography.** Samples from 1% solutions were run in Superdex Peptide column (A₂₃₀ measured).

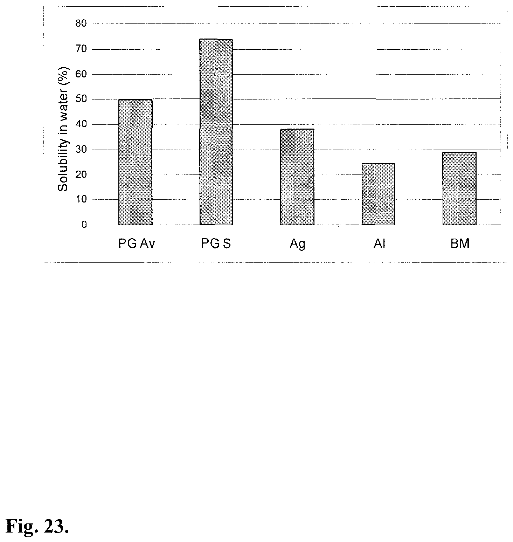

**Fig. 23****.** Solubility of yeast products in water, measured from 1% solutions. PG Av, Progut™ average (n=9); PG S, Progut type Soluble product; Ag, Agrimos; Al, Alphamune™; BM, Bio-Mos®

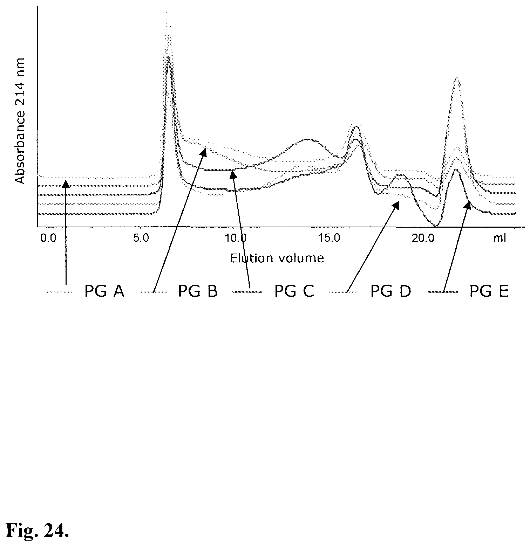

**Fig. 24** shows the analytical GPC analysis of five Progut-type samples. This analysis shows that there is little variation in the GPC profile of the soluble material and thus the production process is well standardized.

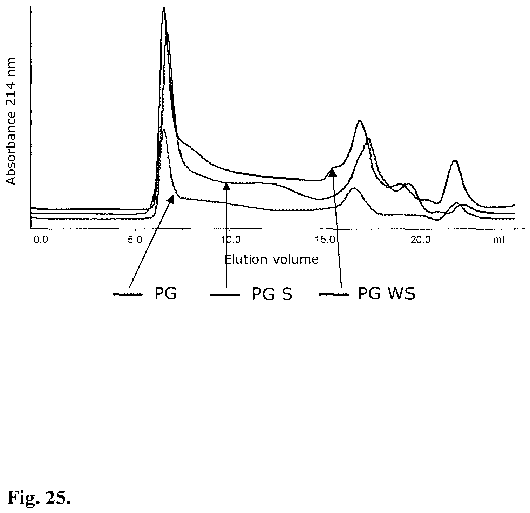

**Fig. 25****.** Analytical GPC analysis of PG, Progut™ ; PG S, Progut type Soluble product; and PG WS, Progut type Water Soluble (totally (water)soluble product, further processed from Progut S).

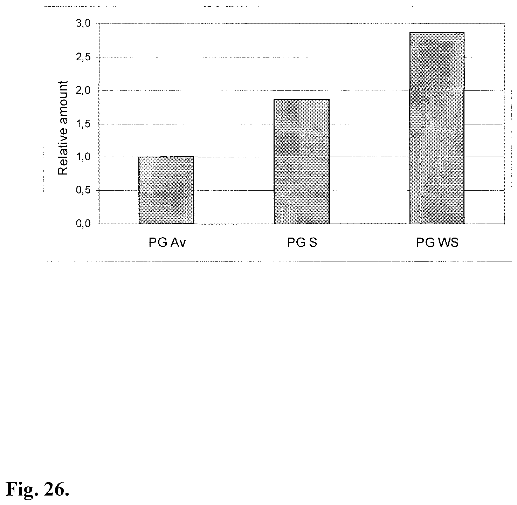

**Fig. 26****.** Comparison of the amounts of soluble poly- and oligosaccharides, as well as peptides. Relative amounts of material were measured as areas from gel-permeation chromatograms at the elution volume 6-14 ml, at 214 nm. PG_{Av} refers to the average area of five different PG batches and is set to 1.

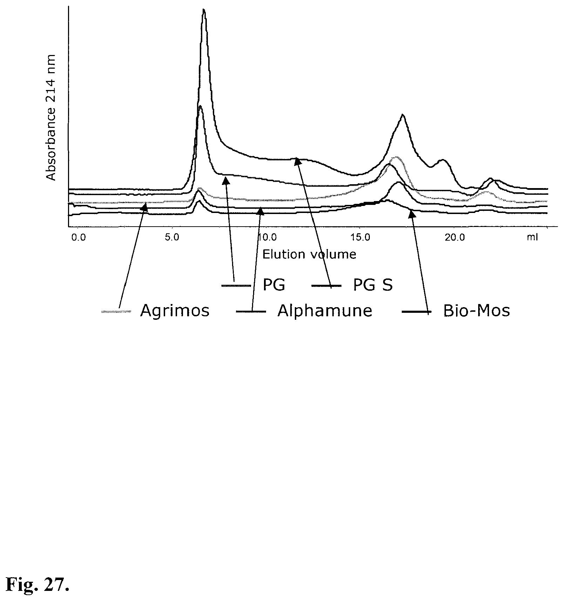

**Fig. 27****.** GPC analysis of PG; PG S; Agrimos; Alphamune™; and Bio-Mos®.

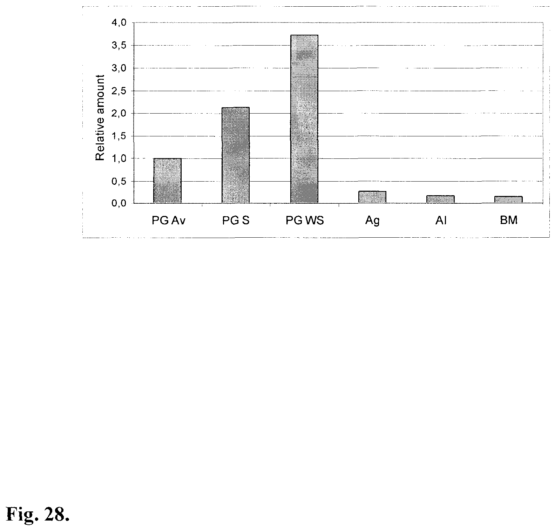

**Fig. 28****.** Comparison of the amount of poly- and oligosaccharides and peptides in Progut-type materials and in three competitor products. PG Av, average (refers to the average area of five different PG batches and is set to 1); PG S; PG WS; Ag, Agrimos; Al, Alphamune™; BM, Bio-Mos®. The results are shown as relative amounts measured from gel-permeation chromatograms, at 230 nm, from 6-14 ml.

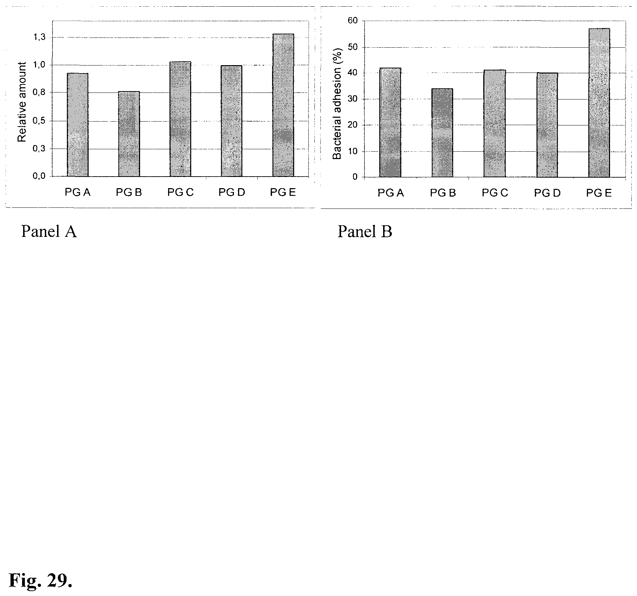

**Fig. 29****.** Correlation of the amount of soluble middle-size material with the bacterial adhesion analysis results of five Progut-type product batches. Panel A. Relative amounts of soluble poly- and oligosaccharide -sized material as measured by GPC (Data from preparative GPC analysis of partially purified i.e. nearly amino acid/peptide free soluble components from 400 mg of 1% PG solutions, measured at 230 nm, from 6-14 ml. Here the average area from five PG samples was 27800 mAu, it was given the value 1). Panel B. *E. coli* bacterial adhesion analysis results.

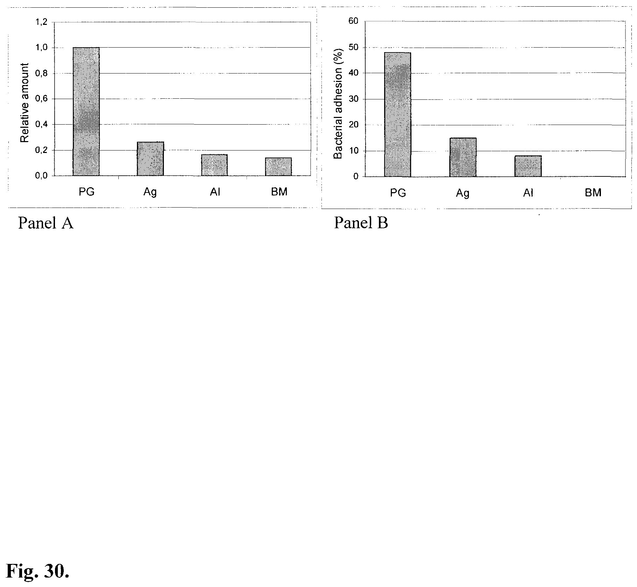

**Fig. 30****.** Correlation of the soluble material amount with the bacterial adhesion analysis results of Progut-type product and competitor samples. Panel A. Relative amounts of soluble poly- and oligosaccharide -sized material. (Data from analytical, 1% sample, GPC analysis of partially purified i.e. nearly amino acid/peptide free soluble components from 400 mg of 1% PG solutions, measured at 230 nm, from 6-14 ml. Here the average area from five PG samples was 205 mAu, it was given the value 1). Panel B. *E. coli* bacterial adhesion analysis results.

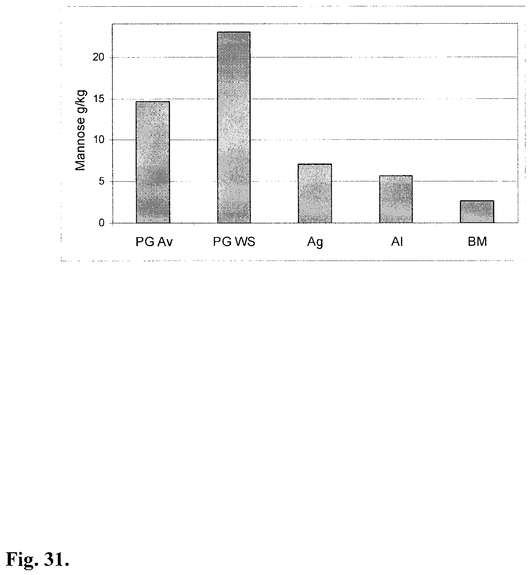

**Fig. 31****.** Mannose content of soluble poly- and oligosaccharide -sized material (i.e. the soluble components from 1% PG or related solutions isolated by GPC, Superdex Peptide 10/30 column, at 6-16 ml). PG Av, average (n=5) ; PG WS; Ag, Agrimos; Al, Alphamune™; BM, Bio-Mos®

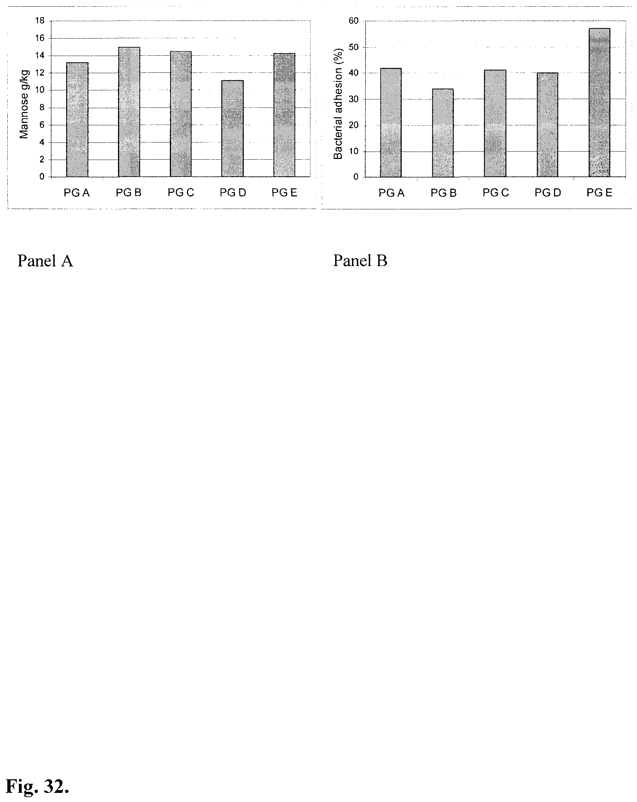

**Fig. 32****.** Comparison of the mannose content with the bacterial adhesion analysis results of five Progut-type product batches. Panel A. Mannose content of soluble poly- and oligosaccharide -sized material (i.e. the soluble components from 1% PG solutions isolated by GPC, Superdex Peptide 10/30 column, at 6-16 ml). Panel B. *E. coli* bacterial adhesion analysis results.

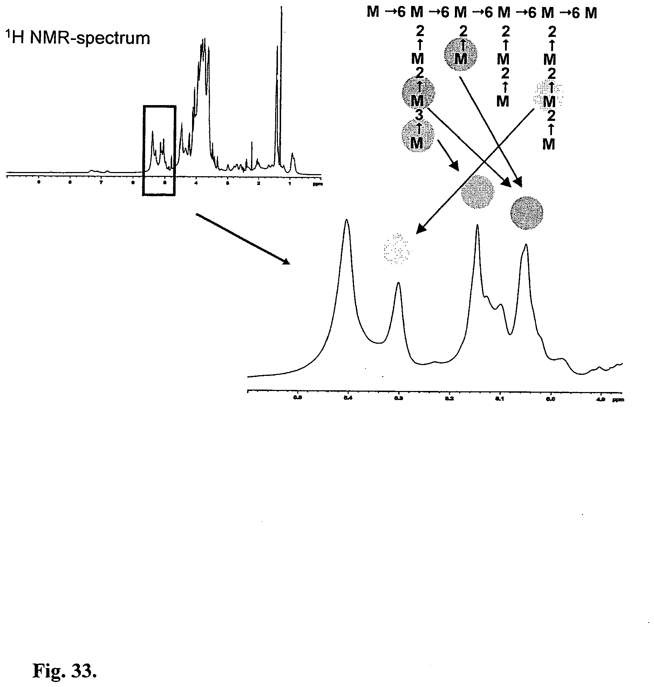

**Fig 33****.** ¹H NMR of soluble poly- and oligosaccharides from Progut-type product. Some structural features of manno-oligosaccharides are indicated from the NMR-spectrum.

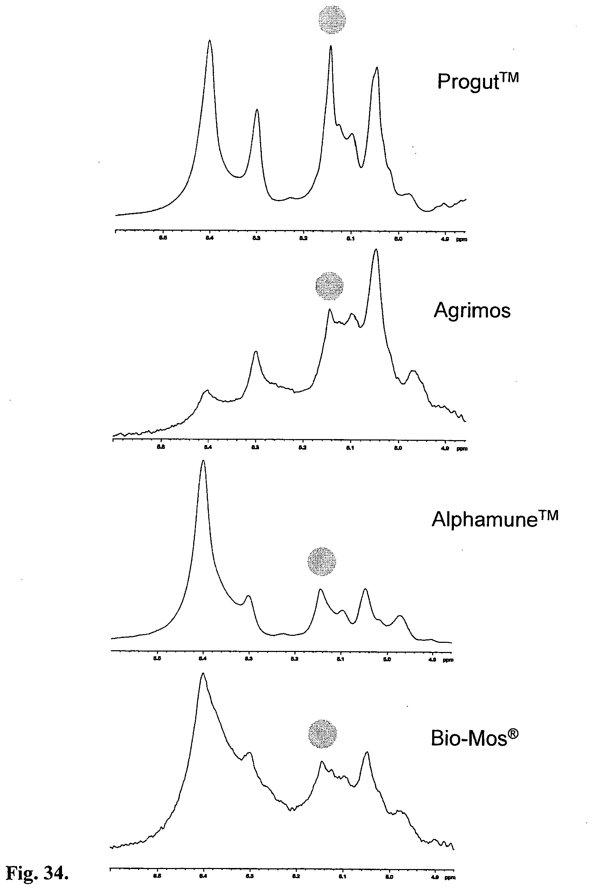

**Fig 34****.** Comparison of ¹H NMR of PG and competitors.

### DETAILED DESCRIPTION OF THE PRESENT INVENTION

### Production of novel soluble brewery yeast derived product

The invention revealed novel methods for producing soluble therapeutic or nutraceutical products (food or feed products or additives thereof) from yeast, preferably brewery yeast type materials. The invention revealed several methods for production of the materials including specific hydrolytic and/or degradative methods
1) specific glycosidase, especially endoglycosidases
2) protein cleaving enzymes such as proteases pronase and savinase,
3) improved chemical hydrolysis especially strong acid hydrolysis, and
4) physical homogenization of the raw material.

The invention is further directed combination of the methods with increasing solubilization activity.

### Referred raw material for producing the novel soluble product

The present invention is preferably directed to the use suitable yeast materials especially yeast form fermentation process, most preferably the brewery yeast as raw material when the yeast has been derived from brewing process. It is realized that such yeast material would include plant derived non-soluble materials, especially plant derived non-soluble materials comprising plant polysaccharides such as hemicellulose saccharides (as shown by cleavage by pectinase) and plant derived β-glucan materials such as cereal beta glucans, preferably the cereal is cereal used in brewing process, most preferably barley. It is realized that the novel enzymatic reactions are useful for the production additional amounts of cereal type soluble saccharides to the novel product.

It is realized that the novel saccharides produced by specific enzymatic reactions or alternatively by chemical methods have additional beneficial health effects.

It is further realized that the preferred soluble combinations can be produced by combining for raw materials
1) yeasts not used in brewing and
2) plant derived polysaccharides preferably plant polysaccharides such as hemicellulose polysaccharides and/or plant glucans especially cereal, and most preferably barley derived polysaccharides or cereal glucans, most preferably barley glucan.

### Preferred process

The preferred optimized process includes at least following steps
1. Providing brewery yeast raw material
2. Hydrolysis by optimized acid hydrolysis or by enzyme and by optimized acid hydrolysis

The invention is further directed to process comprising steps:
1. Providing brewery yeast raw material
2. Physical homogenization of the material
3. Hydrolysis by acid

The invention is further directed to process comprising steps:
1. Providing brewery yeast raw material
2. Physical homogenization of the material
3. Hydrolysis by enzyme and acid

### Novel oligosaccharides and low molecular weight polysaccharide compositions

The novel degradative production processes gave product which has increased solubility or less non-soluble material. Beside the increased solubility the materials have increased biological activities against diarrhea causing infections and activity to increase the growth of animals.

### Isolation of the soluble component from novel product

The invention is especially directed to the separation of the novel soluble fraction produced from the brewery yeast materials.

### Preferred Oligosaccharide compositions in the product

The inventors analyzed the biological activities of the novel soluble products from the profiles of Superdex Peptide chromatograms (1% solutions in water). The absorbance indicates the relative amounts of the soluble different size components.

The relative effectivity of Progut-type product in biological models and related material could be presented in numbers by calculating the elution areas (e.g. Table 1). The invention revealed novel soluble saccharide preparations referred as "Soluble" and "Water soluble" materials useful for the inhition of harmful bacterial activity such as diarrhea causing E. coli activities. It is realized that such preparations are useful for growth and well-being of human and animals.

### Preferred product types

It is realized that the novel hydrolysis process produced novel soluble saccharides which can be used as such as non-fractionated composition derived from the yeast. The preparation is in preferred embodiment neutralized or partly neutralized, preferably by strong alkaline such as sodium hydroxide. In a preferred embodiment the product is dried, e.g by spray dying, drum drying or other known drying methods.

The invention is further directed to fractionated products including partially fractionated and purified saccharide products. Preferred partial purification methods includes removal of part of major non-saccharide components such as 1) desalting, in preferred embodiment by precipitation of salt such as precipitation of phosphate by Calsium or other ions, 2) removal of ionic molecules or hydrophobic molecules by specific adsobents such as hydrophophic or ion exchange matrixes and/or 3) removal of the non-soluble material or part of it. It is realized that the partial purification increase the novel saccharide content of the preparation typically by 10-30 % or even 50 %. Desalting after strong phosphoric acid hydrolysis would increase the amount of saccahrides in preparation enven by 30-60 %, thus increasing the preferred amounts of preferred Manα3Man- mannose saccharides from from about 15, more preferably 20 g/ kg to about 20 g/ke and more preferably 30 g /kg.

Preferred purification methods includes isolation of the saccharide fraction or part of it. The content of the saccharides in the purified fraction is preferably at least 10 % more preferably at least 25 % even more preferably at least about 50 %. The gel filtration fractions measured in NMR are estimated to include saccharide purities of at least 60 %, even more preferably at least 70 %. It is realized that similar purities can be reached by other standard purification and fractionation methods.

### Preferred sizes of the Saccharide materials

The invention revealed two preferred major size ranges of the soluble products large size products eluting from 6 to 12 min from the Superdex Peptide column and intermediate size product eluting from about 12 minutes to about 16 minutes.

The invention is especially directed to the saccharide fractions described, which contains novel useful saccharides and correlate with the improved increased biological activity of the product.

### Preferred soluble polysaccharides sizes and fractions

The product comprises a peak at void corresponding to saccharides with size from about 20 to about 30 Hexose (Hex) units of gel filtration size and to possible larger soluble polysaccharides. More preferably the void from about 7.7 minutes corresponds to soluble polysaccharides about 25 hexose unit saccharides and larger. The invention is directed to novel products comprising high amount of soluble polysaccharide materials, which was a characteristic for the novel products. Preferred polysaccharide fractions includes fraction eluting from 6-9 minutes corresponding to saccharides of about 20 hexose units and larger from the Superdex Peptide column and 6-12 minutes.

It is realized that the elution position at 12 min is about a t border of oligosaccharides (DP10) and polysaccharides comprising more than 10 monosaccharide residues.

The invention is thus directed to the polysaccharide fractions
a)from 10 to 30-mers or gel filtration size from 10 to at least about 30-Hex units and larger soluble polysaccharides
b) from 20 to 30-mers or gel filtration size from 20 to at least about 30-Hex units produced by the processes according to the invention.

### Referred intermediate, size product

The invention is further directed to preferred intermediate size product eluting from about 12 minutes to about 16 minutes. This range corresponds oligosaccharides from about di/trisaccharides to about decasaccharides or saccharides with gel filtration size from 3 to at least about 10-Hex units.

### Preferred larger oligosaccharides and polysaccharide fraction

The invention is further directed to combination fraction of larger oligosaccharides an polysaccharides eluting from about 6 minutes to 14 minutes. This preferred fraction contain oligosaccharides from about 4-6-mers, more preferably from about 5-6-mer to polyasaccharides at least about 20-30 mers or
to gel filtration size from 5 to at least about 30-Hex units and larger soluble polysaccharides.

In gel filtration two hexose (such as glucose, mannose or galactose) units correspond typically to one HexNAc (e.g. GlcNAc or GalNAc) in oligosaccharide or polysaccharide and expected elution size of a saccharide can be calculated typically from the amount of Hex unit.

### Homogenization of the raw material and combination with hydrolysis,

It was revealed that homogenization of brewery yeast solution before process acid hydrolysis increases the solubility of Progut-type product-type product by 15%, with brewery yeast raw material and more acid even more.

The invention is especially directed to processes including homogenization before hydrolysis,in a preferred embodiment acid hydrolysis.

### Enzymatic treatments increases the solubility of acid hydrolysis product (PG)

The invention revealed that product hydrolyzed by acid can be further solubilized by enzymatic treatment with protease or polysaccharide cleaving enzymes. The preferred enzymatic treatment includes Pronase, Savinase, Pectinase, Glucanex and Glucanex combined with savinase or enzymes with specificity for the same protein or polysaccharide type.

### Enzymatic treatments before acid hydrolysis,

The invention revealed that product hydrolyzed by acid is more soluble if enzymatically treated with protease or polysaccharide cleaving enzymes before the acid hydrolysis. The preferred enzymatic treatment includes Pronase, Savinase, Pectinase, Glucanex and Glucanex combined with savinase, and englucanase enzyme optionally combined with savinase or enzymes with specificity for the same protein or polysaccharide type.

### Optimized acid hydrolysis conditions

The invention is directed to optimized acid hydrolysis conditions for producing the novel soluble product.
The invention revealed that acid treatments with increasing amounts of acid increase the solubility to brewery yeast.

The invention is especially directed to optimized hydrolysis processes wherein the amount of the raw yeast material calculated as dry weight is between 5-35 weight/volume (w/v) % of the reaction total volume, more preferably between 10- 30 % (w/v), even more preferably 15 -25 % (w/v) and most preferably about 20 % (w/v) of the reaction total volume.

The present invention is directed to hydrolysis conditions optimized with regard to time, temperature, acid concentration (/amount of acid per amount of sample), and type of acid. Preferred acid includes inorganic acids fosforic acid (H₃PO₄), hydrochloric acid and sulphuric acid. Most preferred acid is fosforic acid (H₃PO₄).

The present invention reveals novel progut type products especially produced with optimized strong acid hydrolysis or enzymatic hydrolysis conditions or surprisingly effective combinations thereof to obtain novel bioactive and soluble product. It was observed that surprisingly strong acid treatment conditions, including very high acid concentrations including about 0.5 M to about 1 M acid and/or high reaction temperature in range of 75- 100 degrees of Celsius, were needed to obtain increased solubilization and obtaining highly bioactive low molecular weight polysaccharides and oligosaccharides. The conditions are drastic and reveal high resistance of yeast materials to hydrolysis, however there is optimum with the conditions so that increase of acid to 2 M at high temperature causes less effective solubilization of the cell wall materials and/or increased amount of free monosaccharides caused by hydrolysis closing to total hydrolysis.

### Optimized hydrolysis by H₃PO₄

The invention is preferably directed to the use of fosforic acid (H₃PO₄) with concentrations above 0.5 M more preferably above about 0,75 M or most preferably with about 1M.
The effect of solubilization was reduced with 2 M fosforic acid even below the effect of 0.5 M, the extreme acid concentration surprising did not increase the hydrolysis but caused precipitation of the material. This indicates that the optimum acid concentration is from about 0.5 M to about 1 M for effective solubilization.

In a preferred embodiment about 1 M H₃PO₄ is used as optimal concentration, in an example the range is from about 0.75 M to about 1.25 M , even from about 0.8 M to about 1.2 M and about 0.9 M to about 1.1 M, when the reaction is performed at temperature about 80 degrees of Celsius (preferably between 70-100 degrees of Celsius, even more preferably between 70 and 95 degrees of Celsius even more preferably between 75 and 85 degrees of Celsius. The preferred reaction time is about 4 hours, preferably from 2 to 8 hours, more preferably 3 to 5 hours, most preferably from 3.5 to 4.5 hours.
The invention is especially directed to strong hydrolysis conditions or combination of strong acid hydrolysis conditions and enzymatic hydrolysis to obtain increased amount of highly soluble and bioactive saccharides according to the invention, in a preferred embodiment hydrolysis condition equivalent of present invention are used.
In a preferred embodiment the conditions are equivalent of about 1 M H₃PO₄ for 4 hours at about 75 to 90 degrees of Celsius for about 4 hours. It realized if temperature is increased the amount of acid and/or reaction time can be decreased and vice versa. In another preferred embodiment the temperature is increased from about 90 to about 100 degrees of Celsius and the amount of acid is from about 0.5 M to about 1 M and the reaction time is about 4 hours. The figure 16 shows high solubilization at 100 degrees with 0.5 and 1 M acid.
It is further realized that the highly soluble and bioactive product can be obtained by using an ezyme or an enzyme in combination with the acid hydrolysis, such as shown in Figure 16 for savinase enzyme. The use of enzyme enhances the solubilization process so that lower acid concentration, reaction temperature or reaction time is needed. The invention is in a preferred embodiment directed to use of an enzyme according to the invention in combination with 0.3- 1 M acid.

### Optimized hydrolysis by hydrochloric acid HCl

The invention is preferably directed to the use of HCl with concentrations above 0.5 M more preferably above about 0.75 M and even more preferably above or about 1M. The 2 M acid produces higher solubilization but also increased amount of monosaccharides as the acid hydrolysis conditions with the very strong mineral acid approaches total hydrolysis conditions.

In a preferred embodiment about HCl is used as optimal concentration, in an example the range is from about 0.75 M to about 1.25 M , even more from about 0.8 M to about 1.2 M and most about 0.9 M to about 1.1 M, when the reaction is performed at temperature about 80 degrees of Celsius (preferably between 70-90 degrees of Celsius, even more preferably between 75 and 85 degrees of Celsius. The preferred reaction time is about 4 hours, preferably from 2 to 8 hours, more preferably 3 to 5 hours, most preferably from a 3.5 to 4.5 hours.

The invention is further directed to use of H₂SO₄ at increased concentrations above 0.5 M and at about 1 M as described above for other acid treatments. The data shows that 2 M acid does not produce increased solubilization, possibly indicating increased precipitation.

### Furthermore

### Combinations of the solubilization methods

The invention further revealed multiple useful combinations of the methods as described in the experimental section. Preferred combinations includes homogenization with enzymatic or chemical hydrolysis.

In a preferred embodiment the invention is directed to the combinations of the methods such as homogenization and improved chemical hydrolysis. This method is preferred to because of high efficiency in solubilization of the product and low costs of the methods and reagents.

The invention further revealed preferred combinations of enzymatic cleavage of proteins and glycosidase cleavage producing high yields of materials. The method is preferably combined with homogenization.

The invention revealed preferred combinations of chemical cleavage methods with enzymatic cleavage of proteins and/or glycosidase cleavage producing high yields of materials. The method is preferably combined with homogenization.

### Improved activities of soluble carbohydrates produced

The invention revealed that the preferred solubilization methods produced increased amounts of soluble molecular materials with useful biological activities. The invention is especially directed to increased activities against pathogenic microbes such as diarrhea causing microbes, especially bacteria including E. coli.

### Preferred enzymatic treatments

### Preferred endoglycosidases

### Cleavage of glucose polysaccharides

The invention revealed that glucose polysaccharide cleaving enzymes are useful in the production of the novel soluble product.

In a preferred embodiment β-linked glucose polysaccharide cleaving endo-β-glucosidase enzymes such as endo-glucosidase and glucanex comprising cellulase activities have useful activities in reducing the insoluble material in the novel production process. The invention is especially directed to cleaving cellulose type and beta-glucan polysaccharides. The preferred β-glucans to be cleaved are Glcβ4Glc-structure comprising β-glucans such as cellulose, cerial b-glucans including β4 and β3-linkages between the glucose residues. The preferred endo-β-glucosidase are endo-glucosidases and cellulases.

The invention is especially directed to the cleavage of residual non-soluble β-glucans such as yeast cell wall glucan, cereal glucan and/or cellulose material in the brewery yeast material, in a preferred embodiment in order to produce higher solubility and/or in order to produce soluble bioactive glucan oligosaccharides.
The invention is especially directed to the production of cellulose and/or cereal β-glucan oligosaccharides comprising structures according to the
Formula

[Glcβ4]ₙGlc-structures,

Wherein n is an integer from 2-20, more preferably 2-10,

The invention is more preferably directed to the production of cereal type glucan oligosaccharide to the product. Preferrably the product comprises β4 and β3-linkages between the glucose residues according to the
Formula

[Glcβ4]ₙ₁[Glcβ3]ₙ₂[Glcβ4]ₙ₃Glcβ3[Glcβ4]ₙ₄Glc-structures,

Whererein n1, n3, and n4 are integers from 0 to about 5, preferably 1 to 4 even more preferably 1 to 3, independently
And
n2 is 0 or 1
An Example is especially directed to the endo-β-glucosidase catalyzed production of the glucan oligosaccharides

The invention revealed presence of α-linked glucose materials in the preferred fractions according to the invention. The invention is further directed to use of endo-α-glucosidases in production of the novel soluble product. The α-glucose polysaccharide cleaving enzymes are especially preferred adjustment and optimization of novel product composition and preferred as additional enzymes with endo-α-glucosidase activity especially endo-α4-glucosidase activity, preferably starch or amylose cleaving enzyme.

### Cleavage of pectin and related hemicellulose materials

The invention is further directed to the treatment of the brewery yeast material by a pectinase enzyme preparation. It is realized that the pectinases have reported to comprise multiple activities. The invention is directed to cleavage of pectin and/or other hemicellulose type materials, such as pectin comprising hemicellulose materials by pectinase enzyme comprising preparations.

In a preferred embodiment the invention is directed to the production of pectin and or hemicellulose derived oligosaccharides or low molecular weight soluble polysaccharides to the product.

### Preferred protease treatments

An example is directed to use of protease to cleave protein materials from the raw material or acid hyrolyzed material. Preferred proteases are savinase or pronase.

### Novel glycan comprising materials with improved solubility,

### Progut type product Soluble and Progut type product Water soluble

The present invention revealed that it is possible to produce an acid hydrolysed yeast preparation with increased water solubility by the optimized methods according to the invention. It is realized, that the highly soluble material is useful for more effective formulation of feed and foods. The soluble fraction is especially preferred for liquid feed and liquid food or food additives, especially drinks and beverages. It is realized that material with much less than 50 % of soluble material are not useful for liquid foods or beverages due to practical reasons, such as bottling and transferring the liquid materials in production, and practical material should preferably have well over 50 % of soluble material. It is realized that some applications would tolerate partially insoluble materials and minor part of insoluble material is in a preferred embodiment used in food compositions as fiber to support nutrition and health. The total solubilities of Progut type product and Soluble version thereof in comparison of some reference products used in animal feed are represented in Figure 23.

The novel soluble products comprise even higher amount of soluble saccharide materials (oligosaccharides and polysaccharides) with improved biological activity. The amounts of soluble poly-and oligosaccharide materials (eluting between 6-14 min from the Superdex Peptide column, as shown in Fig. 25) are represented in Figs. 26 and 28. The amount of oligosaccharides in Progut type product Soluble and Progut type product Water Soluble was about 2-higher and even over 3-times higher, respectively than less optimized Progut type product.

### Preferred solubility ranges of the novel soluble materials

The invention is especially directed to novel Progut type product Soluble, wherein the total solubility as 1 % water solution is over 55 %, more preferably over 60 %, even more preferably over 65 %, even more preferably over 63 %, even more preferably over 66 %, even more preferably over 67 %, and most preferably over 68 %. In a preferred embodiment the water solubility is about 70 %, preferably in range 55 % to 85%, more preferably in range 60 % to 80%, more preferably in range 63 % to 77 %, more preferably in range 65 % to 75%, even more preferably in range 66 % to 74%, even more preferably in range 67 % to 73%.

The invention is further directed to practically completely water soluble saccharide comprising products, wherein the water soluble product has practically complete water solubility as 1 % (weight/volume) water solution at room temperature (preferably between 20-25 degrees of Celsius). The preferred water soluble fraction is isolated from the Progut type product, preferably Progut type product Soluble.
The preferred complete water solubility is at least 90 % or over 90 %, more preferably over 93 %, even more preferably over 95 %, even more preferably over 96 %, even more preferably over 97 %, and most preferably over 98 %.

The invention is further directed to Progut type product Semi Water Soluble, wherein the non-soluble materials are partially removed. It is realized that low partial removal provides material similar to the Soluble product and high removal similar to Water Soluble product. In a preferred embodiment a the non-soluble components may be removed in varying extent from about 10 % to about 80 %, more preferably from about 20 % to about 75 %, more preferably from about 25 % to about to about 66 %, even more preferably from about 33 % to about to about 66 % obtain the Semi Water Soluble product. The total solubility of the Semi Water Soluble product is preferably, within range from about 73 % to about 94 %, more preferably from about 76 % to about 92.5 %, even more preferably from about 80 % to about 90 %, and even more preferably from about 81 % to about 89 %. In a preferred embodiment the solubility is close to about 80 %, preferably within range from about 75 and to about 85 %, or close to about 90 %, within range from about 85 % to about 95 %. The total solubility of the Semi Water soluble fraction is preferably adjusted to cover the solubilities between the Soluble and Water Soluble fraction/materials.
The word about indicates preferably in preferred embodiments within 2 % units of the value, and more preferably within 1%, or more preferably within 0.5 % units from the exact value or in separate embodiment exactly the value.

### Biologically active carbohydrate components

The invention further revealed that novel material with improved solubility has improved biological activity against diarrhea causing bacteria. This is shown in Fig.29 and in comparision to related materials in Fig. 30 panel B. The biological activity correlates with the presence of UV absorbing materials. The materials were deproteinized and hydrophobic impurities were removed and did not contain major impurities in the oligosaccharide range observable by NMR. Therefore the absorbance appears to correspond to the carbohydrate materials, saccharides including water soluble oligo- and polysaccharides, partially due to reducing end aldehyde absorbance and reducing end derivatizations formed in the process and minor amount of GlcNAc residues linked to saccharides. Minor amount of peptide materials are likely included as indicated in figure legends, but based on NMR the quantitative importance of these is limited.

### Active carbohydrate components

### Mannose saccharides

Analysis shown in Fig. 31 reveals that new highly active soluble Progut type products includes increased amounts of mannose oligosaccharides. The Figure 32 shows that the biological activities of the fractions correlates partially with mannose content. The inventors were able to define by NMR-analysis novel highly active mannose oligosaccharides and polysaccharides in Progut type products. The increase of this type of oligosaccharides is especially useful for novel nutraceutical molecules. The mannose content was increased by about 50 % in the novel soluble products.

### Preferred terminal Manα3-structures

The invention revealed further that beside the mannose content the higher activity of the Progut type products (e.g. as shown in Fig. 30) is explained by specific structures of mannose oligosaccharides. The invention is especially directed to oligosaccharide sequences comprising increased amount of terminal Manα3-structures as shown in figures 33-34. The invention further revealed distinct amount of non-reducing end terminal and 3-substitud Manα2-epitopes and midchain Manα2-epitopes in linear Manα2-structures, which are key parts of the mannose saccharides derived from yeast.

The invention is preferably directed to the increased terminal Manα3-structures, more preferably non-reducing end terminal Manα3Manα2 comprising oligosaccharides produced by the process according to the present invention and in a preferred embodiment produced from brewery yeast. In a preferred embodiment the amount of terminal Manα3-structures yields NMR signal, which is larger than the NMR signal of non-reducing end terminal and 3-substitud Manα2-epitopes or larger than the NMR signal of midchain Manα2-epitopes in linear Manα2-structures as indicated in Figures 33-34. In a preferred embodiment the Manα3-signal is higher than either one of the Manα2-epitope signals marked in Fig. 33 in proton NMR spectroscopy as indicated in Figures 33 and 34. The invention is especially directed to the Manα3-glycans, wherein the proton NMR signal at Mana3-position is at least 5 % higher, more preferably at least 7 % higher, even more preferably 9, and most preferably at least 10 % higher that the comparative Manα2-signal indicated in Fig. 33.

It is understood that the terminal non-reducing end Manα3Manα2 structures are novel active components to be directed for inhibition of pathogens, especially diarrheagenic bacteria such as E. coli. The reducing end Manα2-structure gives the glycan novel conformation. The activity of this glycan can not be known based other glycans such as Manα3Manβ4GlcNAc structures or reducing disaccharide epitopes.

The present invention is further directed to the terminal Manα3 enriched saccharide fractions comprising at least about 10 grams, even more preferably at least 15 grams/kg, even more preferably at least 16 grams, even more preferably at least 17 grams, even more preferably at least 18 grams, even more preferably at least 19 grams, even more preferably at least 20 grams, even more preferably at least 21 grams, even more preferably at least 22 grams, and most preferably at least 23 or 24 gram per kg of the preferred mannose oligosaccharides. The preferred mannose oligosaccharide includes oligosaccharides comprising mannose, preferably mainly mannose, but which may further comprise other monosaccharide residues preferably Glc, or GlcNAc or GlcN,preferably as minor components.

### Other active carbohydrate components, special β-glucosaccharide signals

The inventor observed special β-glucose oligosaccharide signals at region of about 4.51-4.53 ppm in proton NMR-spectrum. These are associated novel unusual low molecular weight glucose oligosaccharide and polysaccharide signals. The invention is especially directed to the oligosaccharides enriched with β6-linked glucose saccharide having characteristic signals in the region (gentiobiose H1 of Glcβ6 is at about 4.51 ppm, signals of midchain Glcβ6 residues are at about 4,519; 4,529; and 4.523 ppm; Lo V.M. et al. Carbohydr Res. (1993) 245, 333-345).

The invention is further directed to saccharide materials according to the invention, when the material further comprise minor amount of GlcN/GlcNAc, preferably the materials are Glcβ-materials derived from yeast and more preferably comprise Glcβ6 residues.

The invention is in separate embodiment further directed to β4-linked glucose oligosaccharides, when the yeast raw material is cultivated in (cereal) β3/4glucan or cellulose containing materials, the preferred β(3/)4glucose saccharide signals are in the preferred region (e.g. β3/4glucan oligosaccharides have β4-linked Glc H1 signals at about 4.53 ppm).
The amount of glucose materials is comparable to mannose materials in the sample based on monosaccharide analysis, part of the Glc materials includes Glcα-materials but both alfa and beta signals correspond to substantial amount of saccharides.

### Prefererred enriched oligosaccharides According to the invention

The invention is directed to the preferred saccharides according to the invention, wherein the signals are especially from oligosaccharides due to sharpness of the NMR-signals of the Progut type products. The oligosaccharides according to the invention have degree of oligomerization between 2-10. It is realized that most of the background is directed to polysaccharide materials such as glucans, and large polysaccharides which are insoluble.

### Total saccharide content and larger saccharide content

### Quantitation of oligosaccharides and poly saccharides by UV absorbance

The invention is directed to novel Progut type products with specific larger oligosaccharide and polysaccharide content.

Sample of 0.4 grams of dry Progut type product or relative product was diluted in water as 1 % solution (weight/volume). Sample was centrifuged and water soluble fraction was dried and taken for further analysis. The oligosaccharide and polysaccharide fraction was further purified by removing proteins and peptides and lipophilic impurities by incubating with 1.4 ml Dowex H+-resin and bond elut column (analysis, experimental section).
The purified sample dried in vacuum centrifuge and soluted in 1000 microliters of water. 15 microliter sample of the 1000 micro litres was run in Superdex peptide column and absorbance area from 6 to 14 min was collected at 214 nm. The standardized Progut type product gave 680 mAU (absorbance units) in range of larger saccharides eluting between 6-14 min from the column (containing larger oligosaccharides and polysaccharides).

The invention is specifically directed to novel Progut type products, especially soluble and water soluble product, which give higher than 680 mAU (Abs units, absorbance units) as absorbance integral between 6-14 min per 6 mg of original dry sample, when run in Superdex peptide column (Amersham Pharmacia, Analysis Examples) with flow rate 1.0 ml/min, when the saccharide fraction essentially purified from contaminating peptides and proteins from the saccharide fraction. The invention is also directed to equivalent oligosaccharide fractions with corresponding material size range in gel filtration and material produced by process according to the invention. The preferred values for the absorbance integral are 1.3 times, more preferably 1.5 times, even more preferably 1.7 times, even more preferably 1.8 times, even more preferably 2.0 times, even more preferably 2.3 times, even more preferably 2.5 times, even more preferably 2.7 times, even more preferably 2.8 times, and most preferably preferably 2.9 times, 3.0 times or 3.2 times the 680 mAU.

### Quantitation of oligosaccharides and polysaccharides by Mannose mass

The invention is directed to novel Progut type products with specific total oligosaccharide and polysaccharide content. This was done by analyzing the total mannose content in the oligosaccharide and/or polysaccharide fraction eluting between 6 to 16 min from Superdex peptide column.

### Purification or isolation of soluble saccharide materials

The present invention is especially directed to purified or enriched fractions comprising one or several of the preferred saccharide types according to the invention. It is realized that the enriched or purified fractions have especially high specific activity as nutraceuticals functional foods or feed or nutraceutical (food or feed) additives.

The preferred purification methods include following and combinations thereof
a) chromatographic methods such as
   i. chromatographies for absorption of charged and or lipopholic (hydrophobic impurities)
   ii. size exclusion chromatography, especially gel filtration to remove low molecular weight impurities
   iii. affinity chromatographies with matrices binding to the saccharides or part thereof, preferably chromatography on activated carbon
   and/or
b) Phase separation solution methods such as extraction with solvents and/or precipitation of impurities or saccharides. In a preferred embodiment organic solvent is used for precipitation, preferably an alcohol or ketone such as methanol or ethanol, more preferably ethanol; or acetone is used for precipitation of the saccharides of preferred size.
   and/or
c) Centrifugation and separation of solution and precipitant
   and/or
d) Chemical or enzymatic methods to degrade undesired components, preferably mild alkaline hydrolysis to degrade alkaline labile impurities and/or enzymatic hydrolysis of Glcα-comprising glycogen/starch type saccharides

The invention is further especially directed to the ion exchange and/or hydrophobic chromatographies for removal of impurities and/or size exclusion chromatographies for the purification of the novel saccharide fraction. It is realized that ion exchange in a preferred embodiment cation exchange resin material also absorb lipophilic compounds or both matrixes may be included in the same column.

### Solubility experiments

Figures 9-21 show solubilities product fraction after acid and/or enzyme treatments. The remaining insoluble material in 0.2 % water solutions was measured. The amounts shown corresponds to 100 mg of original dry product in 50 ml. The mg amounts indicated thus correspond to % of insoluble materials. In figure 23 the amount of soluble material is indicated as %.

### EXPERIMENTAL SECTION

### Abbreviations

- BY: Brewery yeast
- HBY: Homogenized brewery yeast
- G: Glucanex
- PG: Progut-type product
- PG S: Soluble Progut-type product
- PG WS: Water-soluble Progut-type product
- PG WS+E: Water-soluble Progut-type product with emulgator
- PE: Pectinase
- PR: Pronase
- S: Savinase

### EXAMPLE 1

### Materials and Methods

### Materials

### Progut™ (PG) samples from Suomen Rehu

Fourteen Progut™ type product samples from different production batches: PG1-PG14. Three Progut-type product samples from different production processes: PG 15h, hydrolyzed for 15 h; PG WS, PG water soluble; PG WS E+, PG water soluble with emulgator.

PG1 has been shown to work well, whereas PG2 was not as effective as indicated on bacterial adhesion assays. PG4 gave good results on the field when tested with goats.

### Commercial samples from other producers

Agrimos (C1), and Ascogen (C2), Alphamune alpharma (C3), and Bio-Mos (C4).

### Whole brewery yeast samples

Brewery yeas, BY and homogenized brewery yeast, HBY.

### Procedure of analysis

### Preparation of the sample

Either 0.2% water solutions or 1% water solutions were prepared from Progut-type product and related samples. These were incubated at room temperature for 2-5 hours under gentle mixing. The mixtures were then centrifuged (4000 rpm for 20 min), the supernatant was lyophilized, and the dry weight was measured.

### Analysis of the soluble components

The soluble components of the samples were purified by two purification steps before gel permeation chromatography: First, the dry material was diluted into a slurry solution of H⁺-beads (AG 50W-X8 resin, Bio-Rad, 1.4 ml for 0.4 g sample) and incubated in gentle mixing at RT for 2 hours. Second, the supernatant from H⁺-bead solution was eluted through BondElut C-18 -column (500 mg / 6 ml, Varian). After concentration the samples were run in gel permeation chromatography in a column of Superdex Peptide 10/300 GL (Amersham Biosciences), with 50 mM ammoniumbicarbonate, at flow rate 1 ml / min, measuring the absorbance at 214 or 230 nm, in Äkta Purifier 10 (Pump P-903, UV-900, Amersham Biosciences). Superdex Peptide column: Void volume, 6.7 ml; total elution volume, 18.0 ml; and elution of poly/oligo/monosaccharides: At void volume 30 Hex units; at 7.7 ml 25 Hex units; at 14.4 ml five Hex units; at 16.7 ml two Hex units, and at 17.3 ml one Hex unit.

### Nuclear magnetic resonance spectroscopy (NMR)

For NMR analysis samples were collected from Superdex Peptide chromatography as follows: Large size components (elution from 6 to 12 ml) and intermediate size components (elution from 12 to 16 ml). Prior to one dimensional ¹H NMR experiments the samples were first lyophilized from and then dissolved in D₂O (99,9%). The NMR spectra were recorded by Varian Unity 500 spectrometer (Varian Inc.) at 23°C.

### Acid hydrolysis of the brewery yeast

The acid hydrolysis of the whole brewery yeast -solution with H₃PO₄ is similar to the hydrolysis used in Progut-type product manufacturing process: pH is adjusted to 2.4 by H₃PO₄ (concentrated, 87%), and incubated at 80°C for four hours. This acid hydrolysis is referred hereinafter as 'process acid hydrolysis' in text.

### Results

### Comparison of the amounts of the soluble components

1% solutions of nine Progut-type product samples and four related samples were prepared and the dry weights of the water-soluble components were measured (Fig. 1). From the results shown in Fig. 1 it can be concluded, that approximately 50% of the Progut-type product material is water-soluble, although the solubility varies depending on the batch. Alphamune alpharma (C3), Bio-Mos (C4), and Agrimos (C1) have less soluble components than Progut-type product. On the other hand, Ascogen (C2) seems to have a good solubility.

However, it should be noticed that in these lyophilized samples may be left variable amounts of water. Therefore, error in dry weights might be higher than on average.

### Comparison of the soluble materials by gel permeation chromatography

### Chromatograms from 0.2% solutions

### Comparison of PG 1 and 2

0.2% solutions of PG1 and PG2 were prepared as described in Materials and Methods, and the chromatograms of the soluble material are shown in Fig. 2. Comparing the soluble materials of PG1 and 2 can be seen that PG1 has relatively more intermediate sized material (elution between 12-16 ml) than PG2 and, on the contrary, PG2 has more large size material (elution between 6-12 ml) than PG1. The overall amount of soluble material seems to be higher in PG1 than PG2 as indicated by the higher absorbtivity in PG1. The same phenomenon can be pointed out also from Fig. 1.

These differences in solubility and chromatographic behavior imply the effectivity of PG1 and PG2: PG1 and PG2 have been tested by bacterial adhesion tests and PG1 was shown to be effective whereas PG2 was ineffective.

### Chromatograms from 1% solutions

### Comparison of PG1 and 2

NMR analysis of the soluble components needs substantial amount of material. Therefore, 1% solutions were produced for semi-preparative gel permeation chromatograms. As a comparison to the chromatograms from 0.2% solutions in Fig. 3 are shown the chromatograms of soluble materials from 1% solutions of PG1 and 2. Although the detector is somewhat overloaded, it is perhaps even more clearly seen that in PG2 there are not as much intermediate sized soluble components as in PG1.

### Comparison of nine Progut-type product and four related samples

Nine Progut-type product (PG1-9) and four related samples (C1-4) were prepared simultaneously and analyzed equally. Therefore, their gel permeation chromatograms are comparable. Here, samples were detected at A₂₃₀, and for proper comparison, PG1 and 2 were reanalyzed. The scale of the absorbance axis is adjusted to be equivalent in all chromatograms in order to facilitate the comparison of the samples.

The chromatograms of PG1, 3, 4, and 6 look rather similar (Fig. 4, A, C, D, F) both in shape and amount of the material. Both PG1 (in bacterial adhesion assay) and PG4 (in field experiment with goats) have been shown to be effective. On the other hand, PG2, 5, 7, and 8 have less material than the other PG samples (Fig. 4, B, E, G, H). From these PG2 is known to be ineffective in bacterial adhesion assay. The data indicates that in a preferred embodiment the effective Progut-type product can be predicted by the shape of the chromatogram and especially by the amount of intermediate size soluble material. The relative amounts of the intermediate size material (eluting at 12-16 ml) can be calculated as area from the chromatogram (mAbs units multiplied by ml) and comparing these values (Table 1) confirms the estimations of the differences between Progut-type product samples. The large size (eluting at 6-12 ml) and the intermediate size (eluting at 12-16 ml) material has been collected for NMR analysis.

In related samples the amounts of intermediate size and even large material is much less than in Progut-type product samples (Fig 4, J, K, L, M). The total amount of soluble material in Alphamune alpharma (C3) and Bio-Mos (C4) (Fig 4, L, M, respectively) looks minor when compared to Progut-type product samples, which can be seen also in Fig. 1. In Agrimos (C1) and especially in Ascogen (C2) the total amount of soluble material is comparable to Progut-type product, but most of it is low molecular weight size material (Fig. 4, J, K). The low molecular weight size material is not good in inhibiting bacterial adhesion, although it might have some other beneficial properties.

**Table 1. Relative amounts of components from chromatograms in Fig. 4. The area was calculated as mAbs units multiplied by ml (here, the areas are combined from three semipreparative chromatograms).**

| **Sample** | **Fig. 4** | **Area** (12-16 ml) | **Area** (6-14 ml) |
|---|---|---|---|
| PG1 | A | 17600 | 22805 |
| PG2 | B | 6400 | 7189 |
| PG3 | C | 14900 | 21685 |
| PG4 | D | 12400 | 22300 |
| PG5 | E | 7700 | 11899 |
| PG6 | F | 10000 | 16429 |
| PG7 | G | 5600 | 9573 |
| PG8 | H | 2800 | 6474 |
| Agrimos | J | 7100 | 2445 |
| Ascogen | K | 14500 | 6724 |
| Alphamune | L | 1100 | 1593 |
| Bio-Mos | M | 2400 | 1384 |

### Comparison of five Progut-type product samples

Progut-type product samples PG10-14 were prepared similarly for gel permeation chromatography as the samples of Fig. 4. Therefore, the chromatograms of figures 4 and 5 are comparable. Although no information of the bioactivities of the PG10-14 is available, these chromatograms imply that these (PG10-14) Progut-type product batches should work well, because in all samples there are substantial amounts of soluble material that largely consists of intermediate to high molecular weight compounds. The large size (eluting at 6-12 ml) and the intermediate size (eluting at 12-16 ml) material has been collected for NMR analysis.

### Acid hydrolyzed brewery yeast and its homogenized version

Brewery yeast solution was homogenized by an industrial pressure homogenizator and samples of both brewery yeast solution and its homogenized version were hydrolyzed by Progut-type product process acid hydrolysis as described in Materials and Methods. In order to isolate samples for NMR by semipreparative gel permeation chromatography the detector was overloaded and chromatograms comparable to Figs 4 and 5 are not available. However, analytical gel permeation chromatograms were run (Fig. 6), and the profiles of the chromatograms mimics that of PG1 (Fig. 2A). Comparison of the areas in the chromatograms of Fig. 6A and B indicates that the homogenization increases solubility by 15%.

### Samples prepared for bacterial adhesion tests

Higher solubility of Progut-type product is needed for dosing it among liquid feed, most preferably in drinking water. Ten sample candidates were chosen aiming to new water-soluble Progut-type product. These samples were prepared in amounts enough for bacterial adhesion tests, their gel permeation chromatograms are presented in Fig. 7. (The experimental processes are presented in Materials and Methods of Example 2.) The samples were:
1. Brewery yeast treated with glucanex and process acid hydrolysis, BY G (Fig. 7 A)
2. Homogenized brewery yeast treated with glucanex and process acid hydrolysis, HBY G (Fig. 7 B)
3. Brewery yeast treated with glucanex and savinase, followed by process acid hydrolysis, BY G+S (Fig. 7 C)
4. Homogenized brewery yeast treated with glucanex and savinase, followed by process acid hydrolysis, HBY G+S (Fig. 7 D)
5. Brewery yeast treated with 1 M H₃PO₄, BY 1M (Fig. 7 E)
6. Homogenized brewery yeast treated with 1 M H₃PO₄, HBY 1M (Fig. 7 F)
7. Brewery yeast treated with glucanex and pectinase, followed by process acid hydrolysis, BY G+PE (Fig. 7 G)
8. Homogenized brewery yeast treated with glucanex and pectinase, followed by process acid hydrolysis, HBY G+PE (Fig. 7 H)
9. PG1 treated with pectinase, PG1 PE (Fig. 7 I)
10. PG1 treated with pronase, PG1 PR (Fig. 7 J)

Five of the candidates for new water-soluble Progut-type product (samples 1, 2, 5, 6, and 9) were finally chosen for bacterial adhesion tests. Treatment of brewery yeast with glucanex and with 1 M H₃PO₄ yielded material with the best activity in bacterial adhesion assay performed similarly as for old Progut-type product testing process.

Interestingly, the chromatograms of brewery yeast and its homogenized version after enzymatic treatment are rather similar. On the contrary, more soluble, intermediate sized material is found in the homogenized brewery yeast treated with 1 M H₃PO₄ than in non-homogenized sample (Fig 7, F versus E).

### Water-soluble Progut-type product

In order to test the effectivity of 1 M H₃PO₄ treated brewery yeast on field, this was produced in industrial scale and the product was named as water-soluble Progut-type product (PG WS). Another version of PG WS was also produced by introducing emulgator, and this was named PG WS+E. Their solubilities were analyzed by gel permeation chromatography and for easy comparison, PG1 was analyzed in the same set of experiments (Fig. 8). When comparing the chromatograms of PG (Fig. 8 A) and PG WS (Fig. 8 B), and relative amounts of material calculated from them (Table 2), it can be concluded that there are over 30% more soluble material in PG WS. Specifically, the relative amount of very large size material (eluting at 6-9 ml) has increased substantially, 60%. However, the relative amount of intermediate size material (12-16 ml) is approximately equal. Hence, the higher concentration of H₃PO₄ in PG WS production appears to break down the insoluble part just enough to make it soluble to water. Although the product is not completely soluble, the change is high enough to allow PG WS to be delivered as liquid feed among drinking water.

Another sample was prepared by a longer (15 hour) hydrolysis (PG 15h) in otherwise normal production process. The gel permeation chromatogram of PG 15h (Fig. 8 D) implies that the product is similar to PG1. NMR analysis will reveal whether there are changes in structures of the components.

**Table 2. Relative amounts of material from chromatograms in Fig. 8A and B. The amount of material is specified as the area from the chromatograms as calculated mAbs units multiplied by ml.**

| **Elution volume** | **PG1** (area) | **PG-WS** (area) | **PG1** (relative amount) | **PG-WS** (relative amount) |
|---|---|---|---|---|
| 6-16 ml | 2454 | 3226 | 1.00 | 1.32 |
| 6-12 ml | 1846 | 2515 | 1.00 | 1.36 |
| 12-16 ml | 607 | 711 | 1.00 | 1.32 |
| 6-9 ml | 1071 | 1727 | 1.00 | 1.61 |
| 9-16 ml | 1383 | 3225 | 1.00 | 1.31 |

NMR-analysis of intermediate size components in Progut-type product samples The most prominent signals in the intermediate size fraction NMR analysis can be assigned to H-1 of α-glycosidic carbohydrate chains. Considering the biological origin of the material analyzed, α-glucan chains and α-mannosidic chains are the expected source of the signals.

The spectra also show signals that originate from H-1 of β-glycosidic units. These signals are expected to originate from various β-glucose residues. Carbohydrate oligomers containing such units are expected to be produced in the process of the present invention from e.g. beta-glucans of yeast and barley.

### Conclusion

Progut-type product samples and related samples from main producers were analyzed for their solubility and their gel permeation chromatography profiles are generated. From these results, it looks like that the relative amount of soluble material of equally processed samples implies the effectivity of Progut-type product in question as animal feed. The amount of both large and intermediate size material seems to be particularly important. In all of the related samples the amount of large and intermediate size material is clearly lower than in Progut-type product samples. In some of the related samples most of the soluble material is of low molecular weight size, which is not likely to be effective in inhibiting bacterial adhesion.

The invention is directed to isolation of the soluble components shown in these gel permeation chromatograms and optional further fractionation of the materials and more detailed chemical analysis e.g. by NMR for the isolated and/ fractionated materials..

The invention is especially directed to the intermediate sized material and its relative amount as important component for activity. The invention is further directed to the large sized material and its relative amount as important component for activity.

### EXAMPLE 2

### Materials and Methods

### Materials

### Progut™ (PG)- type product samples from Suomen Rehu

PG1; PG2; PG 15h, PG hydrolyzed for 15 h; PG WS, PG water soluble; PG WS E+, PG water soluble with emulgator.

PG1 has been shown to work well, whereas PG2 was not so effective on bacterial adhesion assays.

### Whole brewery yeast samples

Brewery yeast samples used in the present experiments had 20% of dry substance, on average.

### Hydrolysis with acid

### Phosphoric acid

The main acid hydrolysis to the whole brewery yeast -solution with H₃PO₄ is similar to the hydrolysis used in Progut-type product manufacturing process: pH is adjusted to 2.4 by H₃PO₄ (concentrated, 87%), and incubated at 80°C for four hours. This acid hydrolysis is referred hereinafter as 'process acid hydrolysis' in text.

Acid hydrolysis with higher concentrations of H₃PO₄ were performed with 0.3 M, 0.5 M, 1 M, and 2 M H₃PO₄ either at 80°C or at 100°C, for four hours.

### Hydrochloric acid

Acid hydrolysis with HCl was performed with 0.3 M, 0.5 M, 1 M, and 2 M HCl at 80°C, for four hours.

### Sulfuric acid

Acid hydrolysis with H₂SO₄ was performed with 0.3 M, 0.5 M, 1 M, and 2 M H₂SO₄ at 80°C, for four hours.

### Solubility experiment

0.2% water solution was prepared either from Progut-type product samples or the brewery yeast -solution (i.e. 500 µl of the reaction mixture, containing 100 mg dry weight material, was diluted up to 50 ml with H₂O This was incubated at room temperature for 2-5 hours under gentle mixing. It was shown that the duration of incubation was not relevant for the solubility. The mixture was centrifuged (4000 rpm for 20 min) and the weight of the precipitate was measured after lyophilization.

### Enzymatic treatments

### Pronase

20 mg of pronase (*Streptomyces griseus*, 48 U / mg, Sigma) for 5 ml of brewery yeast - solution / 20% Progut-type product solution, 5 mM CaCl₂, incubation at 37°C for five hours. The process acid hydrolysis was performed either before or after the enzymatic treatment. The effect of pronase was analyzed by the solubility experiment.

### Savinase

5 ml of brewery yeast -solution / 20% Progut-type product solution, pH adjusted to 10 by NaOH, 4 or 20 µl of savinase (protease from *Bacillus sp*, 16 mU / µl, Sigma), incubation at 37°C for five hours. The process acid hydrolysis was performed either before or after the enzymatic treatment. The effect of savinase was shown by the solubility experiment.

### Glucanex

The effect of glucanex (lysing enzymes from *Trichoderma harzianum*, 1.18 U / g, Sigma) to the solubility of brewery yeast -solution was studied by varying the amount of glucanex or the temperature in the reaction. For 5 ml of brewery yeast -solution 5-100 mg of glucanex was added and incubated at 30, 37 or 50°C for five hours. The process acid hydrolysis was performed after the enzymatic treatment. The effect of glucanex was shown by the solubility experiment.

### Pectinase

20 mg of pectinase was added (*Aspergillus niger*, 1 U / mg, Calbiochem) for 5 ml of brewery yeast -solution or 20% Progut-type product solution and incubated at 37°C for five hours. The process acid hydrolysis was performed either before or after the enzymatic treatment. The effect of pectinase was shown by the solubility experiment.

### Endoglucanase

200 µg of endoglucanase (endoglucanase I from *T. reesei*, from VTT) for 5 ml of brewery yeast -solution, incubation at 37 or 50°C for five hours. This reaction was performed only together with either glucanex or both glucanex and savinase (see below).

### Combined enzymatic treatments

Combined effect of glucanex and savinase: First reaction by glucanex as described, and then savinase at pH 10 as described. The process acid hydrolysis was performed after the enzymatic treatments, after which the effect of the enzymes was shown by the solubility experiment.

Combined effect of glucanex and endoglucanase: Concomitant reactions by glucanex and endoglucanase, at 37 or 50°C, as described. The process acid hydrolysis was performed after the enzymatic treatments, after which the effect of the enzymes was shown by the solubility experiment.

Combined effect of glucanex, endoglucanase, and savinase: First the reaction by glucanex and endoglucanase, at 37 or 50°C as described, and then the savinase at pH 10 as described. The process acid hydrolysis was performed after the enzymatic treatments, after which the effect of the enzymes was shown by the solubility experiment.

### Results

Each series of experiments included a control reaction, which mimicked the production process of the original Progut-type product, i.e. the brewery yeast -solution was treated with the process acid hydrolysis as described in Materials and Methods.

The increase in the solubility of the components after the enzymatic and / or acid hydrolysis experiments were measured inverse fashion: Because the dry weight of the soluble components was rather impossible to measure, the diminishing of the insoluble components was measured instead.

### Effect of pronase

Progut-type product samples were treated with pronase as described in Materials and Methods. PG1 is, according to bacterial adhesion inhibition tests, effective as animal feed and seems to have better solubility than PG2. The solubility of both Progut-type product samples increases considerably by pronase treatment as seen in Fig. 9 where the amounts of insoluble material is presented (the original dry weight material in these samples was 100 mg).

In the next series of experiments the pronase digestions with PG1 and PG2 were repeated, and as shown in Fig. 10, the results are rather similar.

In this series of experiments also the effect of pH in pronase digestion with brewery yeast was studied. Digestions were performed at pH 5 (the pH of intact brewery yeast solution) and at pH 8, and followed by acid hydrolysis. The change in pH during incubation did not cause any significant difference in solubility (data not shown).

The activity of pronase incubated either before or after acid hydrolysis was also studied. The results in Fig. 10 show that pronase is more effective after acid hydrolysis than before it, most probably implying that after acid hydrolysis the proteins are denaturated and more accessible to protease.

Hence, pronase would increase the solubility of Progut-type product even if it is used before acid hydrolysis. Considering the Progut-type product production process, this is beneficial, because the acid hydrolysis would destroy pronase and the product itself would not have enzymatic activity left when used as animal feed.

Comparing the solubility results of Progut-type product or brewery yeast (Fig. 10) it seems that the original, industrial Progut-type product producing process (1) is more effective in producing more soluble product and, (2) this product is more vulnerable to the action of pronase than the lab-scale acid hydrolysis process for intact brewery yeast.

### Effect of savinase

In order to examine the reactivity of another protease with Progut-type product, savinase (protease from *Bacillus sp.*) was chosen, a basic protease which is in industrial production. When tested with Progut-type product, savinase decreased the amount of insoluble material almost in half (Fig. 11). However, the effectivity of pronase (Fig. 10) seems rather better: While pronase diminished the amount of insoluble material up to 22% (PG1), in savinase treated PG1 there was 53% left. In contrast to the action of pronase, the amount of insoluble material is independent whether savinase is used before or after the process acid hydrolysis (cf. Figs 10 and 11, experiments with brewery yeast).

Because savinase is an industrial enzyme, it could be used at reasonable costs. However, the use of savinase is not as practical as the use of pronase: As savinase is a basic protease, the pH has to be adjusted to 10 in order to get effective reaction. This means that after neutralization (regardless whether the savinase is performed before or after the process acid hydrolysis) the sodium phosphate concentration of the product is substantial.

### Effect of pectinase

Pectins are large, heterogeneous, and negatively charged cell-wall polysaccharides. Industrial pectinase is a lysing enzyme of pectin and, in addition, contains several other enzymatic activities as well. Therefore, it was thought that the pectinase treatment could be effective in Progut-type product solution. Indeed, pectinase was shown to be more effective than savinase, but not as effective as pronase in producing more water-soluble Progut-type product (Figs 10, 11, and 12). For example, the dry weight of insoluble material in PG1 diminished with pronase up to 22% and with pectinase to 34%, leaving 8.5 or 15 mg insoluble material, respectively. In contrast to the proteases pronase and savinase, pectinase treatment is effective with brewery yeast solution only after the procedure acid hydrolysis (Fig 12).

### Effect of glucanex

In order to analyze the impact of β-glucans to the solubility of Progut-type product, β-glucanase treatments were performed. Glucanex (lysing enzymes from *Trichoderma harzianum*) was chosen, because it was known as cost-effective yeast cell wall glucan hydrolyzing enzyme. Glucanex is known to contain also cellulase, protease, and chitinase activities. First, the effect of various amounts of glucanex was studied at 30°C. It was clearly shown that small amounts of glucanex did not increase the solubility of brewery yeast substantially. However, with 100 mg / 5 ml of brewery solution, the amount of insoluble material decreased up to 46% as shown in Fig. 13. In the second reaction series the effect of temperature was studied and it was performed with brewery yeast that had been at +4°C for a one month, making it a bit more liquid. Increasing the incubation temperature by 7°C (from 30°C to 37°C) increased the solubility clearly (the insoluble material from 64% to 54%). In contrast, when the temperature was raised to 50°C, the amount of insoluble material decreased only 26%, implying that the enzyme did not tolerate such high temperatures. At 37°C glucanex produces approximately the same reduction in insoluble material amount as pronase and pectinase (cf. Figs 10, 12, and 13).

### Effect of combined enzyme treatments

Protein and polysaccharide degrading enzymes were rather effective in lowering the amount of insoluble material in Progut-type product or brewery yeast solutions, as shown above. However, neither of these enzyme-types was alone able to produce water-soluble product. Next was studied whether the reactivities of proteases and e.g. glucanases are combinatory, i.e. is soluble product producible by combined action of these enzymes.

### Glucanex and savinase

Glucanex and savinase are both cost-effective industrial enzymes, and therefore chosen to be tested for combined reactivity. When comparing the effect of glucanex alone (Fig. 13) or savinase alone (Fig. 11) to their combined action, it is clearly seen that they work more effectively together (Fig. 14): The insoluble material was reduced with glucanex (20 mg, 37°C) treatment alone by 47% and with savinase alone (4 µl, 37°C) by 32%, while in their combined reaction the reduction was 77% (i.e. only 12 mg of original dry weight material was left insoluble). However, even in the best reaction performed (glucanex 100 mg at 30°C and savinase 4 µl at 37°C) there are still 8.5 mg out of the 100 mg dry weight material originally in brewery yeast solution left. Savinase was shown to be less effective than pronase or pectinase and glucanex approximately equally effective as pronase and pectinase (see above). The combined action of glucanex and savinase with brewery yeast solution gives far better results than with pronase and pectinase, and actually resembles the effectivities of pronase and pectinase with Progut-type product.

### Endoglucanase combined with glucanex and/or savinase

In order to improve the polysaccharide degradation, the effectivity endoglucanase (endoglucanase I from *T. reesei*) in combination with glucanex and savinase was studied. Experiments of glucanex and endoglucanase treatment (Fig. 15) and glucanex alone (Fig. 13) show no additive effect; the amounts of insoluble material compared to control are 54% and 53%, respectively. Similarly, with glucanex and savinase, in these experiments the endoglucanase does not improve the solubility (with EG 22% and without 23% of insoluble material versus control).

### Effect of acids

In order to establish the difference between the solubilizing effect of H₃PO₄ compared to other acids, two strong acids, HCl and H₂SO₄, were chosen for acid hydrolysis experiments. The effect of higher acid concentrations to the solubility of brewery yeast solution was also studied.

### H₃PO₄ - effect of increasing concentration with or without savinase

Figure 16 shows the results of two sets of experiments with brewery yeast solution. Clearly, higher concentrations of H₃PO₄ (0.3 M, 0.5 M, and 1 M, at 100°C) increase the solubility of the product (Fig. 16, first three column pairs). Savinase treatment with brewery yeast solution combined with acid hydrolysis at 80°C, in higher concentrations of H₃PO₄ (0.3 M, 0.5 M, 1 M, and 2M) results in rather similar solubility as the acid hydrolysis at 100°C.

### HCl

The acid hydrolysis of brewery yeast solution with variable HCl concentrations (Fig. 17) shows rather similar effectivity as hydrolysis with H₃PO₄.

### H₂SO₄

The acid hydrolysis of brewery yeast solution with increasing H₂SO₄ concentrations (Fig. 18) compared with hydrolysis by H₃PO₄ (Fig. 16) indicates that the acids give fairly similar solubilities. Interestingly, the solubility of the acid hydrolysis product decreases with 2 M acid compared to 1 M. 2 M H₂SO₄ might precipitate components from the brewery yeast solution, because it is such a strong acid.

### Effect of homogenization

In order to analyze whether mechanical treatment of brewery yeast solution would improve the effect of acid hydrolysis, a sample of brewery yeast was homogenized by an industrial homogenizator. There is a 10% increase in solubility when compared to the 1 M acid hydrolysis results of homogenized and normal brewery yeast (Fig. 19, brewery yeast insoluble material 75% left and homogenized brewery yeast 65% left comparing to process hydrolysis). In contrast, as shown in Fig. 20 homogenization has only a minor effect to the degrading effectivity of enzymes glucanex, savinase and pectinase.

### Comparison of PG, PG WS and PG WS E+

According to these solubility results presented here, five samples were chosen to be prepared for bacterial adhesion inhibition experiments. These samples are:
1. Brewery yeast solution with glucanex treatment and process acid hydrolysis,
2. Homogenized brewery yeast solution with glucanex treatment and process acid hydrolysis,
3. 1 M H₃PO₄ acid hydrolysis to brewery yeast solution,
4. 1 M H₃PO₄ acid hydrolysis to homogenized brewery yeast solution, and
5. PG1 treated with pectinase.

They all gave good results in bacterial adhesion assays (performed similarly as in old Progut-type product testing process), glucanex treated sample being the most effective. However, because the 1 M H₃PO₄ acid hydrolysis -product was almost as effective it was chosen to be produced for field experiments.

This water-soluble Progut-type product (PG WS) and water-soluble Progut-type product with emulgator (PG WS+E) were produced in industrial process and samples from them were studied for their solubility. Figure 21 shows the solubilities of water-soluble Progut-type product with and without emulgator in comparison to PG1 and 2. The amount of insoluble material has decreased by 40% when comparing PG1 and PG WS. On the other hand, it seems that the emulgator does not increase the solubility when compared to the solubility of PG WS.

In addition, a sample of overhydrolyzed Progut-type product was brought for analysis. The solubility experiment of this 15 hours hydrolyzed Progut-type product showed that there was almost equal amount of insoluble material left when compared to PG1. Further analysis should be done in order to be able to consider whether there has been e.g. overdegradation.

### Conclusions

The 1 M H₃PO₄ hydrolysis of brewery yeast solution in process production produces a product that can be delivered to animals as a component of drinking water. This means that the amount of insoluble material left in the water-soluble Progut-type product (26%) is small enough that it will stay in liquid during the delivery process to animals. This procedure producing the PG-WS has increased the solubility remarkably, from 42% of insoluble material of current Progut-type product product to 26% of PG-WS.

Enzymatic treatments combined with process acid hydrolysis of either Progut-type product or brewery yeast solution produced even more soluble products than above mentioned 1 M H₃PO₄ hydrolysis alone. At the best the amount of insoluble material was diminished to 10% when treated with pronase or combined action of glucanex and savinase.

### EXAMPLE 3

### Solubility of Progut-type product, novel soluble product and competitors in water

The hydrolysis process was developed further to produce Progut type soluble product, referred as Progut type Soluble, which can be used in animal feeding as liquid mixtures in automated feeders without delivery problems. The solubility of Progut-type product is on average about 50% (n=9) while the Progut type Soluble product has a solubility over 70%. The competing products all show solubilities less than 40% (Fig. 23).

### Features of the Progut type Soluble product

Concerning the health and well-being of the animals, the biologically relevant materials in Progut-type product and other yeast products are thought to be soluble poly- and oligosaccharides. Here, in 1% or more diluted solutions all oligosaccharides are soluble. The insoluble part consists of cell wall components, i.e. primarily of insoluble polysaccharides and proteins. In gel-permeation chromatography (GPC) the soluble poly-and oligosaccharides as well as peptides (also referred to in this report as "middle-size material") elute at 6-14 ml, and their relative amounts are measured at 214 nm.

The modified hydrolysis process of Progut type Soluble product yields almost 2-fold increase in the middle-size material and the amount detected in the further processed novel Progut type Soluble product is almost three times higher (Fig. 25 and 26). The invention also revealed isolated practically totally soluble product referred as Progut type product Water Soluble.

The amount of poly- and oligosaccharides and peptides is substantially lower in three competitor products (Fig. 27 and 28).

### Correlation of the soluble material with bacterial adhesion analysis

The biological activity of Progut-type product is proportional to the amount of middle-size material. Fig. 29 shows that a higher amount of poly- and oligosaccharides and peptides leads to a higher activity in bacterial adhesion. It is also evident that the competitor products carrying low amounts of middle-size material show much lower *E. coli* bacterial adhesion (Fig 30).

### Monosaccharide analysis - comparison of the mannose content

Mannans and mannooligosaccharides in yeast products are believed to be bioactive components. The mannose content of the soluble poly- and oligosaccharide fraction was therefore measured and as shown in Fig. 31, Progut-type product products have clearly more soluble mannooligosaccharides than competitive products.

The mannose content was also compared to the *E. coli* bacterial adhesion activity. It was found that the correlation between the mannose amount and the bacterial adhesion activity is not straightforward (Fig. 32). It is therefore hypothesized that not all mannose poly- and oligosaccharides show similar bacterial adhesion activity, but certain structures show higher bioactivity.

### NMR analysis of soluble components

¹H NMR analysis is able to reveal structural characteristics of mannooligosaccharides in Progut-type product and other yeast products. Fig. 33 shows the NMR analysis of Progut-type product sample. The structural details of a typical yeast mannan saccharide and their characteristic NMR peaks are shown in the inset.

By comparing the NMR spectra of Progut-type product and the three competitor products (Fig. 34), it can be concluded that the relative amount of terminal Manα1-3 units (blue marks in Figs 33 and 34) is clearly higher in Progut-type product. Some *E.coli* strains (harmful to gut health) have been shown to bind preferentially to Manα1-3 structures in mammals. The increase of oligosaccharides with terminal Manα1-3 units is a consequence of the Progut-type product process hydrolysis step.

### Details of the 1H NMR analysis

The NMR samples have been divided into two categories according to their saccharide composition.

Low molecular weight Mannopolysaccharide/mannose oligosaccharide containing samples The lineshapes/widths of saccharide signals suggest that the saccharides are fairly large. The anomeric 1H signals of the constituent mannose units have been assigned based on the published data for higher mannan oligosaccharides. The following manno-oligosaccharide fragments have been identified. The mannose unit corresponding to the capital letter is bolded. The relative concentration of the different mannose residues in each sample is given in Table 1. The integration was performed using peak heights and the signal for A was given the value 1. According to integration, the concentration of a terminal α-D-Manp-(1-3)-α-D-Manp-(1-2) and other common mannose epitopes is higher in PG samples compared to the samples from competitors, furthermore the relative proportion of α-D-Manp-(1-3)-α-D-Manp-(1-2) epitope B to internal α-D-Manp-(1-2) epitope A is is higher in the. All of these samples also contain starch as indicated (+) in Table 3. The integration of key Mannose signal indicates that the starch content is smaller in the PG type samples.

List of specific monosaccharide structures corresponding to mannose NMR signals A-E
A) -α-D-Manp-(1-2)-α-**D-Manp-**(1-2)-α-D-Manp-
B) **α-D-Manp-**(1-3)-α-D-Manp-(1-2)-α-D-Manp-
C) 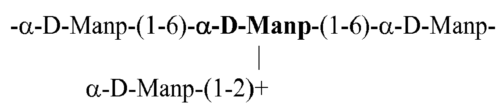
D) -α-D-Manp-(1-2)-**α-D-Manp**-(1-6)-α-D-Manp-
E) -α-D-Manp-(1-6)-**α-D-Manp-**(1-2)-α-D-Manp-
   and 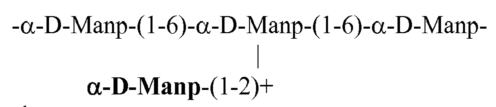 and
   α-D-Manp-(1-3)-**α-D-Manp**-(1-2)-α-D-Manp-

**Table 3. ¹H-NMR signals of mannooligosaccharide fragments and the relative concentrations of different mannose residues. In the name of the sample 'large' indicates large size components from the gel permeation chromatography, eluting at 6-12 ml.**

| NMR sample | Starch | Manno-oligosacharide fragments | | | | |
|---|---|---|---|---|---|---|
| | | A | B | C | D | E |
| | | 5.302 ppm | 5.144 ppm | 5.118 ppm | 5.096 ppm | 5.048 ppm |
| PG1 large | + | 1 | 1.5 | 0.9 | 0.8 | 1.3 |
| PG2 large | + | 1 | 1.4 | 0.8 | 0.7 | 1.3 |
| PG4 large | + | 1 | 1.3 | 1 | 0.8 | 1.3 |
| PG 15h large | + | 1 | 1.5 | 1 | 1 | 1.5 |
| PG S large | + | 1 | 1.3 | 1 | 1 | 1.4 |
| PG WS large | + | 1 | 1.4 | 1.1 | 1.1 | 1.6 |
| PG S +E large | + | 1 | 1.4 | 0.8 | 0.8 | 1.4 |
| PG10 large | + | 1 | 1.4 | 1 | 1 | 1.5 |
| PG11 large | + | 1 | 1.3 | 0.9 | 0.8 | 1.3 |
| PG12 large | + | 1 | 1.4 | 0.9 | 0.8 | 1.4 |
| PG13 large | + | 1 | 1.6 | 0.8 | 0.8 | 1.5 |
| PG14 large | + | 1 | 1.7 | 0.9 | 0.9 | 1.5 |
| Agrimos large | + | 1 | >1^{x} | 1.3 | 1.4 | 2.1 |
| Ascogen large | + | 1 | 1.1 | 1 | 1 | 1.2 |
| Alphamune large | + | 1 | 1.1 | 0.8 | 0.8 | 1.1 |
| Bio-Mos large | + | 1 | 0.9 | 0.9 | 0.8 | 1 |

| | | | | | | |
|---|---|---|---|---|---|---|
| ^{x}difficult to assign due to background but clearly smaller than 5.302 | | | | | | |

### Starch oligosaccharide containing samples

The following samples contain starch oligosaccharides as the only identifiable saccharide component: PG1 intermediate (eluting from GPC at 12-16 ml), PG2 intermediate, PG3 intermediate, PG4 intermediate, PG6 intermediate, PG7 intermediate, PG8 intermediate, PG9 intermediate, PG 15h intermediate, PG10 intermediate, PG11 intermediate, PG12 intermediate, PG13 intermediate, PG14 intermediate, Ag intermediate, As intermediate, Al intermediate, and BM intermediate.

The assignment is based on the presence of the signals at 5.409 ppm, 4.972 ppm, 5.231 ppm and 4.645 ppm, corresponding to the anomeric H1s of [α-(1-4)Glcp]ₙ -chain, α-(1-6)-Glcp branch point and the reducing end α-D-Glcp and β-D-Glcp, respectively.

There are also several samples which contain possibly mannooligosaccharide fragments in addition to the starch oligosaccharide main component. Identification of these molecules requires further investigation.

In NMR spectra of samples PG1 intermediate, PG S intermediate, PG S+E intermediate, and PG WS intermediate signals corresponding to terminal fragments α-D-Manp-(1-3)-α-D-Manp-(1-2)-α-D-Manp- and α-D-Manp-(1-2)-α-D-Manp-(1-2)-α-D-Manp- were present.

### Low starch amounts

Table 4 includes quantitation of starch in the large saccharide fraction by NMR. In a preferred embodiment the invention is directed to saccharide fractions (6-12 min and optionally also oligosaccharides) and material according to the invention, wherein the amount of starch Glcα4 epitopes is less than 3.5 fold more that of Manp-(1-3)-α-D-Manp-(1-2), more preferably less than 3 fold. The invention further revealed presence reducing end signals of starch in the present samples indicating low molecular weight starch chains. Furthermore the preferred starch according to invention contains modest amounts of Glcα6-branch structures. The invention is directed to low amounts of starch in context of high amounts of the preferred mannose saccharides. It is realized that the starch fragments can be degraded by amylase type enzymes. In a preferred embodiment the residual starch material is degraded by starch degrading enzymes such as amylase to obtain more pure mannose/glucose saccharides. The analysis of intermediate Superdex peptide gel filtration fraction reveals that the amount of starch oligosaccharides is particularily low in the novel soluble fractions (PG Water soluble and Soluble).

### Presence of higher amounts of mannose oligosaccharides in the new soluble preparations

The signals of mannose oligosaccharides were more prominent in the soluble (Soluble and Water Soluble) saccharide preparations. When comparing the α-D-Manp-(1-3)-α-D-Manp-(1-2)-α-D-Manp- signal to starch oligosaccharide signal between soluble preparations these signals were at least about 20 % of starch Glcβ4 signal, in certain preparations even about 30-60 % of the starch signal and about the same amount of total assignable oligosaccharides. In old process preparations only very minor α-D-Manp-(1-3)-α-D-Manp signals were observed and these were only a few %, typically less than about 5 % of the starch signal. The intermediated fraction comprising larger oligosaccharides eluting between 12-16 min comprises most of the oligosaccharides except the smallest ones (disaccharides and probably part or trisaccharides) but not monosaccharides. It is realized, that presence of smaller mannose oligosaccharides is not likely in the samples. The amount of the intermediate fraction (superdex peptide 12-16) is substantial part of the total saccharide type material as indicated by the gel filtration profiles, estimated to be about 20-50 % of total saccharides. Therefore the samples include at least about 4 % -30 %, more preferably at least about 5- 30 %, even more preferably at least about 10-30 % mannose oligosaccharides of the total saccharides. The amount of mannose oligosaccharides of the total mannose comprising saccharides is about 10-60 % more preferably 20-60 % based on the mannose and glucose content of the sample.

The mannose oligosaccharides in the preparations are highly enriched with the terminal Manα3-epitope, which is the major mannose signal in the preparations, indicating enrichment of the novel bioactive mannose saccharide inhibiting mannose binding pathogens.

The data reveals that the actual major mannose oligosaccharides with lower molecular weight eluting between 12-16 minutes are effectively produced by the present optimized method to produce soluble , but not other methods including previous progut methods or by the competing method.
Surpringly the competitor materials appears not to contain significant amounts of mannose oligosaccharides, though some of these are advertised as manno-oligosaccharides. It appears that effective production of mannose oligosaccharides from yeast is not easy task despite the efforts of many companies.

The residual mannose materials of competitors appear as high molecular weight poorly soluble materials as demonstrated also by the very broad and weak NMR signals in comparision to more narrow and smooth signals of the materials according to the present invention. The invention is in a preferred embodiment directed to novel saccharide material according to the invention producing NMR spectrum with essentially similar with regard to the mannose signals A-E and Glcbeta-signals according to the invention, including preferably similar peak width, and /or similar peak integrals and heights and similar low amounts of starch signals and/or other impurities such as impurity signal at about 4.7 ppm including possible non-carbohydrate material.

### References for NMR analysis:

Kath, F., and Kulicke, W-M (1999) Die Angewandte Makromoleculare Chemie 268, 69-80 Shibata, N., Kojima, C., Satoh, Y., Satoh, R., Suzuki, A., Kobayashi, H., and Suzuki, S. (1993) Eur. J. Biochem 217, 1-12
Ikuta, K., Shibata, N., Blake J.S., Dahl M.V., Nelson, R.D., Hisamichi, K., Kobayashi, H., Suzuki, S., and Okawa Y. (1997) Biochem J. 323, 297-305

In conclusion, Progut-type product contains more terminal Manα1-3 units as shown by ¹H NMR (Figs 33 and 34) and the mannooligosaccharide content is higher as shown by the monosaccharide analysis of soluble material (Fig. 31) than in competitor products. According to these results can be concluded that the amount of active mannooligosaccharides is clearly higher in Progut-type product than in competitive products.

### Assignment of alfa- and beta-Glc comprising materials

The Table 4 shows specific presence Glcb materials in Progut type samples. The highest amount of Glcb material was found from the Soluble product indicated by NMR signal at about 4.53 ppm. The invention is especially directed to novel Soluble products when the amount of Glcβ NMR signal at about 4.53 ppm is at least about 80 % (0.8 times in Table 4) of Manα3 signal at about 5.14 ppm, more preferably 90 % (0.9 times in Table 4) of the Manα3 signal. The invention is directed to even equimolar amounts of terminal
Glcβ and Manα3- materials.
It is notable that none of the competing materials contain the Glcβ-signal. The Glcβ-signal is considered to include major amount of Glcβ6-material, this is further supported by presence of the signal assignable to Glcβ6-H2 signal at 3.31 ppm. The Glcβ-signal also overlaps the region for cerial glucan signal and signal shapes considered to indicate partial presence of Glcβ4 epitope of cerial glucan derived saccharides in the preferred saccharide material.

**Table 4. ¹H-NMR signals of oligosaccharide fragments and the relative concentrations of different monosaccharide residues. In the name of the sample 'large' indicates large size components from the gel permeation chromatography, eluting at 6-12 ml.**

| NMR sample | Oligosaccharide fragments | | | | | |
|---|---|---|---|---|---|---|
| | Manα1-3 | Glcβ1-6^{a} | Glcβ1-3^{b} | Glcα1-4 | Glcα1-6 | Glcβ1-6^{c} |
| | H1 | H1 | H1 | H1 | H1 | H2 |
| | 5.144 | 4.53^{d} | 4.76 | 5.409 | 4.972 | 3.31 |
| PG1 large | 1 | 0.7 | + | 1.7 | 0.3 | yes |
| PG S large | 1 | 0.9 | + | 3.0 | 0.6 | yes |
| PG10 large | 1 | 0.6 | + | 2.7 | 0.6 | yes |
| PG11 large | 1 | 0.4 | n.d. | 1.7 | 0.6 | yes |
| PG12 large | 1 | 0.3 | - | 1.2 | 0.3 | yes |
| PG13 large | 1 | 0.3 | n.d. | 1.9 | 0.5 | yes |
| PG14 large | 1 | 0.4 | - | 1.0 | - | yes |
| Agrimos large | 1 | - | - | 0.5 | 0.6 | no |
| Ascogen large | 1 | - | - | 3.6 | 1.5 | no |
| Alphamune large | 1 | - | - | 8.0 | 0.1 | no |
| Bio-Mos large | 1 | - | - | 4.0 | - | no |

| | | | | | | |
|---|---|---|---|---|---|---|
| ^{a} Note: H1 signals of Glcβ1-6 and Glcbβ1-4 have the same value. ^{b} H1 of Glcβ1-3 branch in Glcβ1-6 glucan ^{c} The intensity of this signal was not measured, instead is referred whether it is detected (yes) or not (no) ^{d} Note. There is rather large background at 4.53 ppm; therefore, the intensities might be somewhat overestimated + small signal, clearly detectable doublet, too small to be integrated - no detectable signal n.d. not determined, the signal arising from H₂O overlaps the possibility to detect this signal | | | | | | |

### EXAMPLE 4. Animal trials

The novel saccharide preparation PG Water Soluble is fed to test animals as food supplement with amounts of 0.3% -3 % of the feed or drinking water using standard methods used in testing Progut (published by Suomen Rehu). Higher weight gains and/or less diarrhea is observed than with regular Progut preparations or competing products.

## Claims

1. Method for producing saccharide comprising product for use in the prevention of gastric disorders, intestinal diseases and the promotion of growth in animals or human comprising:
a. Providing yeast raw material, preferably brewery yeast raw material
b. Hydrolysis by optimized acid hydrolysis by inorganic acid selected from the group HCl, phosphoric acid or sulphuric acid, in concentration of 0.5 M to 1 M and at the reaction temperature in range of 75-100 °C and the reaction time is from 2 to 8 hours; or by enzyme and optimized acid hydrolysis by inorganic acid selected from the group HCl, phosphoric acid or sulphuric acid, in concentration of 0.5 M to 1 M and at the reaction temperature in range of 75-100 °C and the reaction time is from 2 to 8 hours to yield saccharide composition, wherein the total solubility of the composition is over 55 % as 1 % solution in water.

2. The method according to claim 1, wherein wherein both enzymatic hydrolysis and optimized acid hydrolysis are used.

3. The method according to any of claims 1 or 2, wherein the enzymatic treatment includespronase, savinase, pectinase and/or endoglucanase or includes enzymes with specificity for the same protein or polysaccharide type.

4. The method according to claim 1 comprising steps:
a. Providing yeast raw material, preferably brewery yeast raw material
b. Physical homogenization of the material
c. Hydrolysis by inorganic acid.

5. The method according to claim 4 wherein
the reaction time is 3 to 5 hours or from 3.5 to 4.5 hours.

6. A saccharide comprising product for use in the prevention of gastric disorders, intestinal diseases and the promotion of growth in animals or human obtainable by process according to any of the claims 1-5, wherein the total solubility of the composition is selected from the group over 55 %, between 60 % and 80 % or above 70 %, or over 90 % as 1 % solution in water.

7. The saccharide comprising product according to claim 6, wherein the composition comprises increased amount of saccharides eluting between about 6 to 16 ml and/or 6 to 14 ml form Superdex Peptide 10/300 GL column with total elution volume of 18 ml and column length 30 cm.

8. The saccharide comprising product according to the claim 7, wherein the composition comprises increased amount of Manα3Manα2-oligosaccharides.

9. The method according to any of the claims 1-5, wherein the soluble saccharide fraction is isolated or purified by a method or/ any combinations thereof selected from the group:
a. chromatographic methods such as
i.chromatographies for absorption of charged and or lipopholic (hydrophobic impurities)
ii. size exclusion chromatography, especially gel filtration to remove low molecular weight impurities
iii. affinity chromatographies with matrices binding to the saccharides or part thereof, preferably chromatography on activated carbon
and/or
b. Phase separation solution methods such as extraction with solvents and/or precipitation of impurities or saccharides
and/or
c. Centrifugation and separation of solution and precipitant
and/or
d. Chemical or enzymatic methods to degrade undesired components, preferably mild alkaline hydrolysis to degrade alkaline labile impurities and/or enzymatic hydrolysis of Glcα-comprising glycogen/starch type saccharides.

10. The method according to claim 9, wherein the organic solvent used for precipitation of impurities or saccharides is an alcohol or ketone such as methanol or ethanol, more preferably ethanol or acetone is used for precipitation of the saccharides of preferred size.

11. The saccharide comprising product according to claim 8, wherein there is amount of Manα3Manα2-mannose oligosaccharides being at least about 10 % of total Manα3Manα2-mannose saccharides eluting between 12-16 min.

12. The saccharide comprising product according to claim -8 or 11, wherein there is amount of Glcβ-saccharides so that the integral H1 proton NMR signal of Glcβ at about ppm 4.53 is at least 80 % of Manα3Manα2- H1 proton NMR signal.

## Patentansprüche

1. Verfahren zur Herstellung von Saccharid-haltigem Produkt zur Verwendung bei der Prävention von Magenerkrankungen, Darmerkrankungen und Förderung des Wachstums von Tieren und Menschen, umfassend:
a. Bereitstellen von Heferohstoff, bevorzugt Bierheferohstoff
b. Hydrolyse durch optimierte saure Hydrolyse durch anorganische Säure, ausgewählt aus der Gruppe HCl, Phosphorsäure oder Schwefelsäure, mit einer Konzentration von 0,5 M bis 1 M und bei der Reaktionstemperatur im Bereich von 75-100 °C, und die Reaktionszeit beträgt 2 bis 8 Stunden; oder durch enzymatische Hydrolyse und optimierte Säurehydrolyse durch anorganische Säure, ausgewählt aus der Gruppe HCl, Phosphorsäure oder Schwefelsäure, mit einer Konzentration von 0,5 M bis 1 M und bei der Reaktionstemperatur im Bereich von 75-100 °C, und die Reaktionszeit beträgt 2 bis 8 Stunden, um eine Saccharidzusammensetzung zu erhalten, wobei die Gesamtlöslichkeit der Zusammensetzung als 1%-ige wässrige Lösung über 55 % liegt.

2. Verfahren nach Anspruch 1, wobei sowohl eine enzymatische Hydrolyse als auch eine optimierte saure Hydrolyse verwendet werden.

3. Verfahren nach einem der Ansprüche 1 oder 2, wobei die enzymatische Behandlung Pronase, Savinase, Pektinase, und/oder Endoglucanase beinhaltet oder Enzyme mit Spezifität für das gleiche Protein oder den gleichen Polysaccharidtyp beinhaltet.

4. Verfahren nach Anspruch 1, umfassend die Schritte:
a. Bereitstellen von Heferohstoff, bevorzugt Bierheferohstoff
b. Physikalisches Homogenisieren des Materials
c. Hydrolyse durch anorganische Säure.

5. Verfahren nach Anspruch 4, wobei
die Reaktionszeit 3 bis 5 Stunden oder 3,5 bis 4,5 Stunden beträgt.

6. Saccharid-haltiges Produkt zur Verwendung bei der Prävention von Magenerkrankungen, Darmerkrankungen und der Förderung des Wachstums von Tieren oder Menschen, erhältlich durch den Prozess nach einem der Ansprüche 1-5, wobei die Gesamtlöslichkeit der Zusammensetzung als 1%-ige wässrige Lösung ausgewählt ist aus der Gruppe über 55 %, zwischen 60 % und 80 %, oder über 70 %, oder über 90 %.

7. Saccharid-haltiges Produkt nach Anspruch 6, wobei die Zusammensetzung eine erhöhte Menge an Sacchariden umfasst, die aus einer Superdex Peptide 10/300 GL Säule mit einem Gesamtelutionsvolumen von 18 ml und einer Säulenlänge von 30 cm zwischen etwa 6 bis 16 ml und/oder 6 bis 14 ml eluieren.

8. Saccharid-haltiges Produkt nach Anspruch 7, wobei die Zusammensetzung eine erhöhte Menge von Manα3Manα2-Oligosacchariden umfasst.

9. Verfahren nach einem der Ansprüche 1-5, wobei die lösliche Saccharidfraktion durch ein Verfahren oder Kombinationen von Verfahren abgetrennt oder gereinigt werden, ausgewählt aus der Gruppe:
a. chromatographische Verfahren, wie
i. Chromatographien für Absorption geladener und/oder lipophiler (hydrophober Verunreinigungen)
ii. Größenausschlusschromatographie, insbesondere Gelfiltration zum Entfernen niedermolekularer Verunreinigungen
iii. Affinitätschromatographien mit einer Matrix, die sich an Saccharide oder Teile davon bindet, bevorzugt Chromatographie an Aktivkohle
und/oder
b. Phasentrennungs-Lösungsverfahren, wie Extraktion mit Lösungsmitteln und/oder Ausfällung von Verunreinigungen oder Sacchariden
und/oder
c. Zentrifugierung und Trennung von Lösung und Fällungsmittel und/oder
d. chemische oder enzymatische Verfahren, um unerwünschte Komponenten abzubauen, bevorzugt schonende alkalische Hydrolyse zum Abbau alkalisch instabiler Verunreinigungen und/oder enzymatische Hydrolyse von Glcα-haltigen Sacchariden vom Glykogen-/Stärke-Typ.

10. Verfahren nach Anspruch 9, wobei das zum Ausfällen von Verunreinigungen oder Sacchariden verwendete organische Lösungsmittel ein Alkohol oder Keton ist, beispielsweise Methanol oder Ethanol, besonders bevorzugt wird Ethanol oder Aceton zum Ausfällen der Saccharide von bevorzugter Größe verwendet.

11. Saccharid-haltiges Produkt nach Anspruch 8, wobei eine Menge an Manα3Manα2-Mannose-Oligosacchariden von mindestens etwa 10 % der gesamten der Manα3Manα2-Mannosesaccharide vorliegt, die zwischen 12 und 16 Minuten eluiert.

12. Saccharid-haltiges Produkt nach Anspruch 8 oder 11, wobei eine Menge an Glcβ-Sacchariden vorliegt, sodass das bei etwa 4,53 ppm vorliegende integrierte NMR-Signal des H1-Protons von Glcβ eine Größe von mindestens 80 % des NMR-Signals des Manα3Manα2-H1-Protons hat.

## Revendications

1. Procédé pour la production d'un produit comprenant des saccharides en vue de l'utilisation dans la prévention de troubles gastriques, de maladies intestinales et dans la stimulation de la croissance chez les animaux ou les êtres humains, comprenant :
a. se procurer une matière première à base de levure, de préférence une matière première à base de levure de brasserie ;
b. effectuer une hydrolyse par hydrolyse acide optimisée par un acide inorganique choisi dans le groupe consistant en HCl, acide phosphorique ou acide sulfurique, à une concentration de 0,5 M à 1 M et à la température de réaction dans la plage de 75-100°C et le temps de réaction est de 2 à 8 heures ; ou par une hydrolyse enzymatique et une hydrolyse acide optimisée par un acide inorganique choisi dans le groupe consistant en HCl, acide phosphorique ou acide sulfurique, à une concentration de 0,5 M à 1 M et à la température de réaction dans la plage de 75-100°C et le temps de réaction est de 8 à 8 heures pour produire une composition de saccharides, la solubilité totale de la composition étant de plus de 55 % en tant que solution à 1 % dans l'eau.

2. Procédé selon la revendication 1, dans lequel à la fois une hydrolyse enzymatique et une hydrolyse acide optimisée sont utilisées.

3. Procédé selon l'une quelconque des revendications 1 ou 2, dans lequel le traitement enzymatique comprend de la pronase, de la savinase, de la pectinase et/ou de l'endoglucanase ou comprend des enzymes ayant une spécificité pour le même type de protéine ou de polysaccharide.

4. Procédé selon la revendication 1, comprenant les étapes consistant à :
a. se procurer une matière première à base de levure, de préférence une matière première à base de levure de brasserie ;
b. homogénéisation physique de la matière ;
c. hydrolyse par un acide inorganique.

5. Procédé selon la revendication 4, dans lequel le temps de réaction est de 3 à 5 heures ou de 3,5 à 4,5 heures.

6. Produit comprenant des saccharides pour une utilisation dans la prévention de troubles gastriques, de maladies intestinales et dans la stimulation de la croissance chez les animaux ou les êtres humains, pouvant être obtenu par un procédé selon l'une quelconque des revendications 1-5, la solubilité totale de la composition étant choisie dans le groupe de plus de 55 %, entre 60 % et 80 % ou au-dessus de 70 %, ou au-dessus de 90 % en tant que solution à 1 % dans l'eau.

7. Produit comprenant des saccharides selon la revendication 6, dans lequel la composition comprend une quantité accrue de saccharides s'éluant entre environ 6 et 16 ml et/ou 6 et 14 ml à partir d'une colonne Superdex Peptide 10/300 GL avec un volume d'élution total de 18 ml et une longueur de colonne de 30 cm.

8. Produit comprenant des saccharides selon la revendication 7, dans lequel la composition comprend une quantité accrue de Manα3Manα2-oligosaccharides.

9. Procédé selon l'une quelconque des revendications 1-5, dans lequel la fraction saccharidique soluble est isolée ou purifiée par un procédé ou toutes combinaisons de ceux-ci choisis dans le groupe :
a. procédés chromatographiques tels que
i. chromatographies pour l'absorption d'impuretés hydrophobes chargées et/ou lipophiles ;
ii. chromatographie d'exclusion stérique, en particulier filtration sur gel pour retirer des impuretés de faible masse moléculaire ;
iii. chromatographies d'affinité avec des matrices se liant aux saccharides ou à une partie de ceux-ci, de préférence chromatographie sur charbon actif ;
et/ou
b. procédés en solution à séparation de phases, tels qu'extraction par des solvants et/ou précipitation d'impuretés ou de saccharides ;
et/ou
c. centrifugation et séparation de solution et de précipitant
et/ou
d. procédés chimiques ou enzymatiques pour dégrader des composants indésirables, de préférence hydrolyse alcaline douce pour dégrader des impuretés labiles alcalines et/ou hydrolyse enzymatique de saccharides de type glycogène/amidon comprenant Glcα.

10. Procédé selon la revendication 9, dans lequel le solvant organique utilisé pour la précipitation d'impuretés ou de saccharides est un alcool ou une cétone, tel que le méthanol ou l'éthanol, de manière davantage préférée l'éthanol ou l'acétone est utilisé pour la précipitation des saccharides de dimension préférée.

11. Produit comprenant des saccharides selon la revendication 8, dans lequel il existe une quantité de Manα3Manα2-mannose oligosaccharides qui sont d'au moins environ 10 % des Manα3Manα2-mannose saccharides totaux s'éluant entre 12-16 minutes.

12. Produit comprenant des saccharides selon l'une des revendications 8 ou 11, dans lequel il existe une quantité de Glcβ-saccharides de telle sorte que le signal de RMN du proton H1 intégral de Glcβ à environ ppm 4,53 est au moins 80 % du signal de RMN du proton Manα3Manα2-H1.
